# EUROPÄISCHE PATENTSCHRIFT

(11) **EP 2 576 547 B1**
(45) Veröffentlichungstag und Bekanntmachung des Hinweises auf die Patenterteilung: **27.01.2016**
(21) Anmeldenummer: 11720806.6
(22) Anmeldetag: 24.05.2011
(51) Int. Cl.: C07D 471/04, A61K 31/437, A61P 9/00, A61P 15/00

(54) **SUBSTITUIERTE 5-FLUOR-1H-PYRAZOLOPYRIDINE UND IHRE VERWENDUNG**
SUBSTITUTED 5-FLUORO-1H-PYRAZOLOPYRIDINES AND USE THEREOF
5-FLUORO-1H-PYRAZOLOPYRIDINES SUBSTITUÉES ET LEUR UTILISATION

(30) Priorität: 26.05.2010 DE 102010021637
(43) Veröffentlichungstag der Anmeldung: 10.04.2013
(73) Patentinhaber: Adverio Pharma GmbH, 12529 Schönefeld (DE)
(72) Erfinder: FOLLMANN, Markus, 42489 Wülfrath (DE); STASCH, Johannes-Peter, 42651 Solingen (DE); REDLICH, Gorden, 44799 Bochum (DE); ACKERSTAFF, Jens, 10437 Berlin (DE); GRIEBENOW, Nils, 41541 Dormagen (DE); KROH, Walter, 42115 Wuppertal (DE); KNORR, Andreas, 40699 Erkrath (DE); BECKER, Eva-Maria, 42327 Wuppertal (DE); WUNDER, Frank, 42117 Wuppertal (DE); LI, Volkhart Min-Jian, 42553 Velbert (DE); HARTMANN, Elke, 42111 Wuppertal (DE); MITTENDORF, Joachim, 42113 Wuppertal (DE); SCHLEMMER, Karl-Heinz, 42113 Wuppertal (DE); JAUTELAT, Rolf, 42781 Haan (DE); BIERER, Donald, 42781 Haan (DE)
(86) Internationale Anmeldenummer: PCT/EP2011/058431
(87) Internationale Veröffentlichungsnummer: WO 2011/147809

(56) Entgegenhaltungen:
- WO-A1-00/06569
- WO-A1-98/16223
- WO-A1-03/095451

## Beschreibung

Die vorliegende Anmeldung betrifft neue substituierte 5-Fluor-1H-Pyrazolopyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Innenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *ini-vivo* Eigenschaften, wie beispielsweise ihrem pharmakokinetischem und pharmakodynamischem Verhalten und/oder ihrer Dosis-Wirkungsbeziehung.

Gegenstand der vorliegenden Erfindung sind Verbindungen der allgemeinen Formel (I) 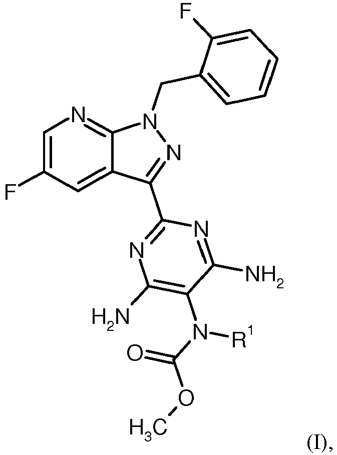 in welcher
- R¹: für Wasserstoff oder (C₁-C₄)-Alkyl steht,
wobei (C₁-C₄)-Alkyl mit ein oder zwei Substituenten unabhängig voneinander ausgewählt aus der Gruppe Fluor und Trifluormethyl substituiert sein kann,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren *N*-Oxide, Salze, Solvate und Solvate der *N*-Oxide und Salze, die von Formel (I) umfassten Verbindungen der nachfolgend genannten Formeln und deren *N*-Oxide, Salze, Solvate und Solvate der *N*-Oxide und Salze sowie die von Formel (I) umfassten, nachfolgend als Ausführungsbeispiele genannten Verbindungen und deren *N*-Oxide, Salze, Solvate und Solvate der *N*-Oxide und Salze, soweit es sich bei den von Formel (I) umfassten, nachfolgend genannten Verbindungen nicht bereits um *N-*Oxide, Salze, Solvate und Solvate der *N*-Oxide und Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl.
Halogen steht im Rahmen der Erfindung für Fluor, Chlor, Brom und Iod.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt im Rahmen der vorliegenden Erfindung sind Verbindungen der Formel (I), in welcher
- R¹: für Wasserstoff oder Methyl steht,
wobei Methyl mit einem Substituenten Trifluormethyl substituiert sein kann,
sowie ihre Salze, Solvate und Solvate der Salze.

Besonders bevorzugt im Rahmen der vorliegenden Erfindung sind folgende Verbindungen der Formel (I):
Methyl-{4,6-diamino-2-[5-fluor-1-(2-fluorbenzyl)-1H-pyrazolo[3,4-b]pyridin-3-yl]pyrimidin-5-yl}carbamat
Methyl-{4,6-diamino-2-[5-fluor-1-(2-fluorbenzyl)-1H-pyrazolo[3,4-b]pyridin-3-yl]pyrimidin-5-yl}methylcarbamat
Methyl-{4,6-diamino-2-[5-fluor-1-(2-fluorbenzyl)-1H-pyrazolo[3,4-b]pyridin-3-yl]pyrimidin-5-yl}(2,2,2-trifluorethyl)carbamat
Methyl-{4,6-diamino-2-[5-fluor-1-(2-fluorbenzyl)-1H-pyrazolo[3,4-b]pyridin-3-yl]pyrimidin-5-yl}carbamat-Hydrochlorid
Methyl-{4,6-diamino-2-[5-fluor-1-(2-fluorbenzyl)-1H-pyrazolo[3,4-b]pyridin-3-yl]pyrimidin-5-yl}carbamat-Sulfat
Methyl-{4,6-diamino-2-[5-fluor-1-(2-fluorbenzyl)-1H-pyrazolo[3,4-b]pyridin-3-yl]pyrimidin-5-yl}carbamat-Phosphat
Methyl-{4,6-diamino-2-[5-fluor-1-(2-fluorbenzyl)-1H-pyrazolo[3,4-b]pyridin-3-yl]pyrimidin-5-yl}carbamat-Mesylat
Methyl-{4,6-diamino-2-[5-fluor-1-(2-fluorbenzyl)-1H-pyrazolo[3,4-b]pyridin-3-yl]pyrimidin-5-yl}carbamat-Ethan-1,2-disulfonat
Methyl-{4,6-diamino-2-[5-fluor-1-(2-fluorbenzyl)-1H-pyrazolo[3,4-b]pyridin-3-yl]pyrimidin-5-yl}carbamat-Maleat
Methyl-{4,6-diamino-2-[5-fluor-1-(2-fluorbenzyl)-1H-pyrazolo[3,4-b]pyridin-3-yl]pyrimidin-5-yl}carbamat-Nitrat.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man die Verbindung der Formel (II) 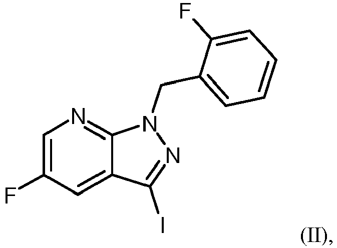
[A] in einem inerten Lösungsmittel in Gegenwart von Hexabutylzinn und einem geeigneten Palladiumkatalysator unter intermediärer Bildung einer Zinnspecies mit der Verbindung der Formel (III) 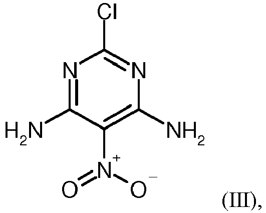 zur Verbindung der Formel (IV) 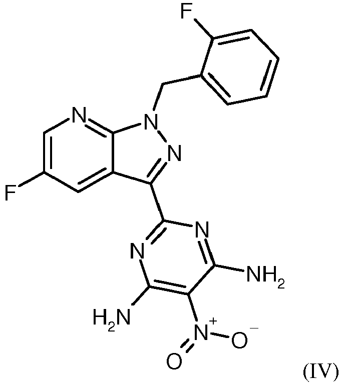 umsetzt, diese dann in einem inerten Lösungsmittel mit einem geeigneten Reduktionsmittel zur Verbindung der Formel (V) 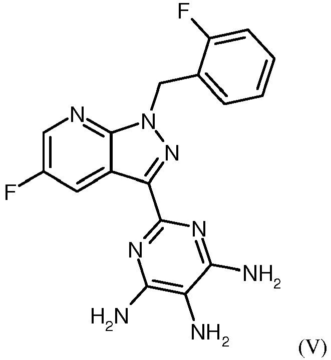 reduziert, diese im Anschluss in Gegenwart einer geeigneten Base mit oder ohne Lösungsmittel mit Chlorameisensäuremethylester zur Verbindung der Formel (I-A) 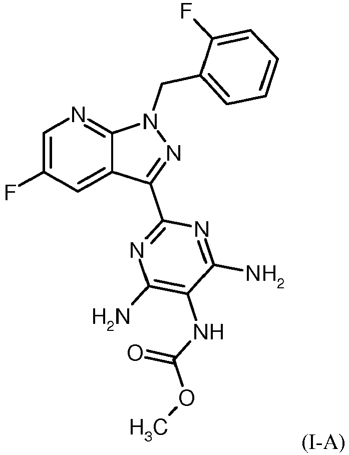 umsetzt,
   oder
[B] die Verbindung der Formel (II) in einem inerten Lösungsmittel mit Kupfercyanid zur Verbindung der Formel (VI) 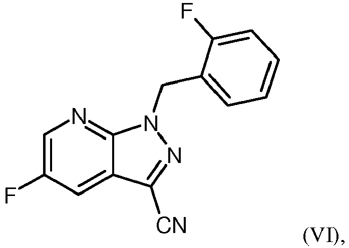 umsetzt, diese dann unter sauren Bedingungen in die Verbindung der Formel (VII) 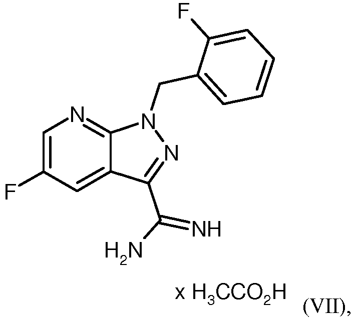 überführt, diese im Folgenden in einem inerten Lösungsmittel in Gegenwart einer geeigneten Base mit der Verbindung der Formel (VIII) 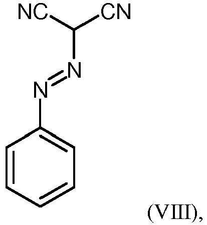 zur Verbindung der Formel (IX) 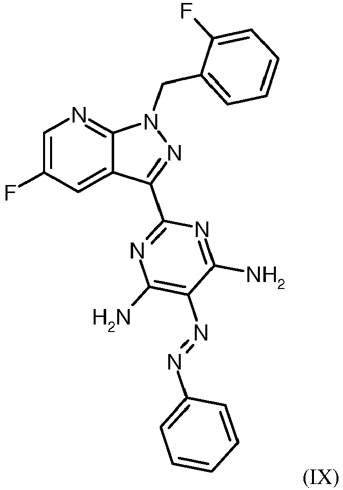 reagiert, und diese anschliessend in einem inertem Lösungsmittel in Gegenwart eines geeigneten Reduktionsmittels zur Verbindung (V) reduziert, und diese im Folgenden nach Verfahren [A] weiter zu Verbindung (I-A) umsetzt,
   oder
[C] die Verbindung der Formel (I-A) in einem inerten Lösungsmittel in Gegenwart einer geeigneten Base mit einer Verbindung der Formel (X) 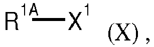 in welcher
   - R^{1A}: für (C₁-C₄)-Alkyl steht,
   wobei (C₁-C₄)-Alkyl mit ein oder zwei Substituenten unabhängig voneinander ausgewählt aus der Gruppe Fluor und Trifluormethyl substituiert sein kann,
   und
   - X¹: für eine Abgangsgruppe, wie beispielsweise Halogen, insbesondere Brom oder Iod, Trichlormethansulfonat, Mesylat oder Tosylat steht
zu einer Verbindung der Formel (I-B) 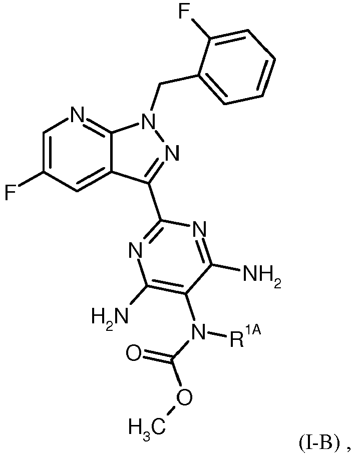 in welcher R^{1A} die oben angegebene Bedeutung hat,
umsetzt,
gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

Inerte Lösungsmittel für den Verfahrensschritt (II) + (III) → (IV) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N'*-Dimethylpropylenharnstoff (DMPU), Dimethylacetamid, *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist Dioxan.

Als Palladium-Katalysator für den Verfahrensschritt (II) + (III) → (IV) ist beispielsweise Palladium auf Aktivkohle, Palladium(II)-acetat, Tetrakis-(triphenylphosphin)-palladium(0), Bis-(triphenylphosphin)-palladium(II)-chlorid, Bis-(acetonitril)-palladium(II)-chlorid und [1,1'-Bis(diphenylphosphino)ferrocen]dichlorpalladium(II)-Dichlormethan-Komplex, gegebenenfalls in Verbindung mit zusätzlichen Phosphanliganden wie beispielsweise (2-Biphenyl)di-*tert*.-butylphosphin, Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] geeignet. Bevorzugt wird Tetrakis-(triphenylphosphin)-palladium(0) verwendet.

Die Reaktion (II) + (III) → (IV) wird im Allgemeinen in einem Temperaturbereich von +20°C bis +180°C, bevorzugt bei +50°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Reduktionen (IV) → (V) und (IX) → (V) erfolgen in Gegenwart eines geeigneten Katalysators in einem inerten Lösungsmittel, in einem Temperaturbereich von +20°C bis +40°C unter Wasserstoff-Normaldruck.

Inerte Lösungsmittel für die Reduktionen (IV) → (V) und (IX) → (V) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt sind DMF und Pyridin.

Geeignete Katalysatoren für die Umsetzungen (IV) → (V) und (IX) → (V) sind beispielsweise Palladium auf Aktivkohle, Platin auf Kohle, Palladiumhydroxid oder Raney-Nickel.

Die Reduktionen (IV) → (V) und (IX) → (V) können alternativ mit einem Metall bzw. Metallsalz wie beispielsweise Eisen, Zink oder Zinn(II)chlorid in einer geeigneten Säure wie beispielsweise Chlorwasserstoff/ Salzsäure, Schwefelsäure, Phosphorsäure oder Essigsäure in einem Temperaturbereich von +20°C bis +140°C erfolgen.

Inerte Lösungsmittel für den Verfahrensschritt (V) → (I-A) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*.*N'*-Dimethylpropylenharnstoff (DMPU), *N*-Methylpyrrolidon (NMP), Acetonitril oder auch Wasser. Ebenso ist es möglich, Gemische der genannten Lösungsmittel einzusetzen. Bevorzugt sind Dimethylformamid und Toluol sowie ein Gemisch aus Dimethylformamid und Toluol.

Geeignete Basen für den Verfahrensschritt (V) → (I-A) sind Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]-non-5-en (DBN). Bevorzugt ist Pyridin.

Die Reaktion (V) → (I-A) wird im Allgemeinen in einem Temperaturbereich von -10°C bis +30°C, bevorzugt bei 0°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (II) → (VI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N'*-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (II) → (VI) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (VI) → (VII) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in Essigsäure unter Bildung des Amidins (VII) bei +50 bis +150°C.

Inerte Lösungsmittel für den Verfahrensschritt (VII) + (VIII) → (IX) sind Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*.*N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Geeignete Basen für den Verfahrensschritt (VII) + (VIII) → (IX) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (VII) + (VIII) → (IX) wird im Allgemeinen in einem Temperaturbereich von +20°C bis +150°C, bevorzugt bei +80°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindung der Formel (VIII) kann analog Literatur L.F.Cavalieri, J.F.Tanker, A.Bendich, J.Am.Chem.Soc., 1949, 71, 533 dargestellt werden.

Inerte Lösungsmittel für die Umsetzung (I-A) → (I-B) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*.*N'*-Dimethylpropylenharnstoff (DMPU), *N*-Methylpyrrolidon (NMP), Pyridin, Acetonitril. Bevorzugt ist Tetrahydrofuran.

Geeignete Basen für den Verfahrensschritt (I-A) → (I-B) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Lithium-bis-(trimethylsilyl)amid, Natrium-bis-(trimethylsilyl)amid und Natriumhydrid.

Die Reaktion (I-A) → (I-B) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die beschriebenen Herstellverfahren können durch die folgenden Syntheseschemata (Schema 1 bis 3) beispielhaft verdeutlicht werden:

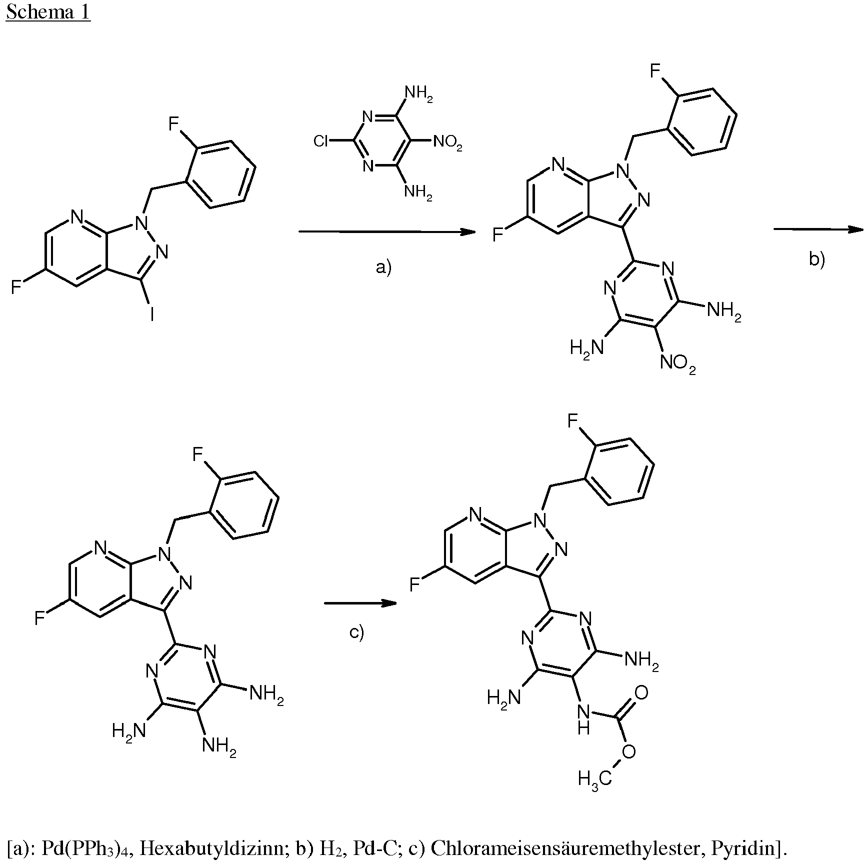

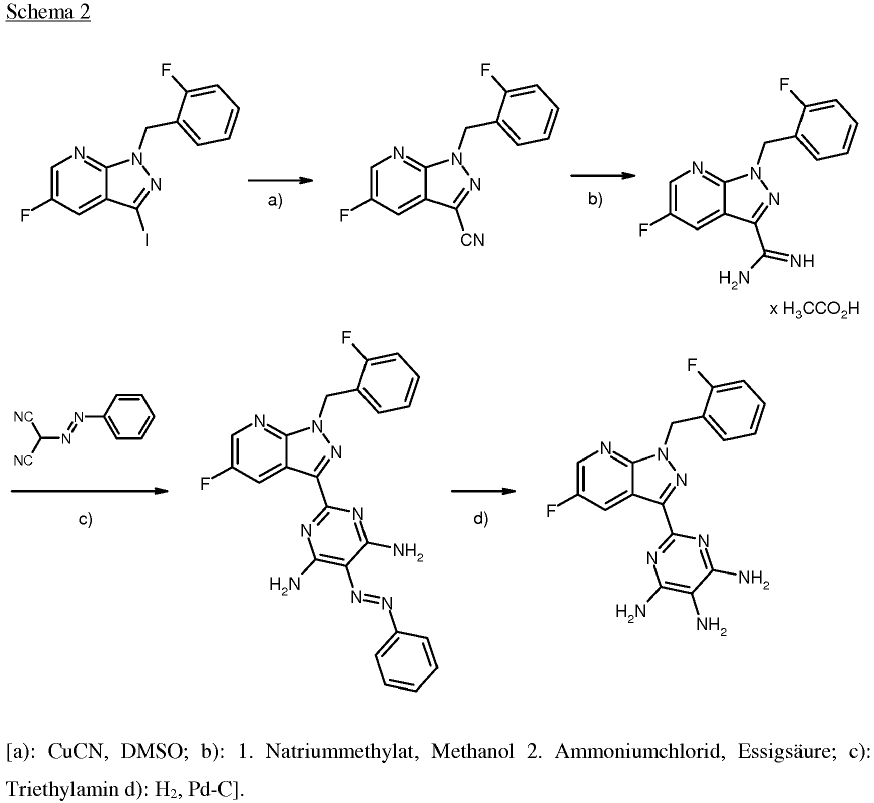

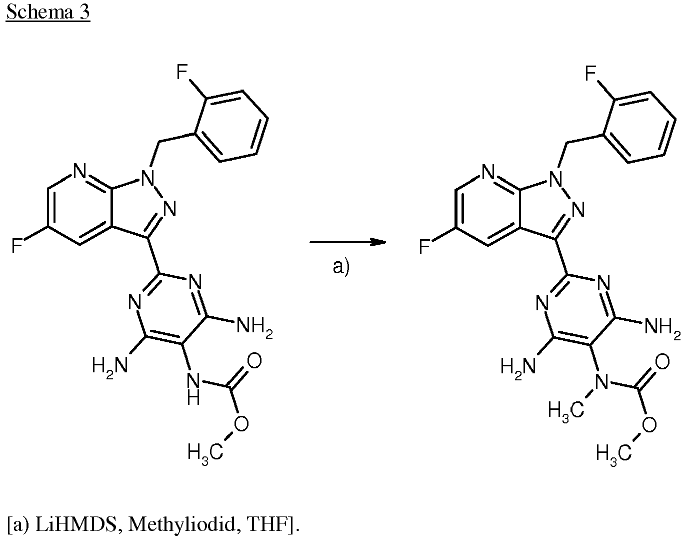

Die Verbindung der Formel (II) kann hergestellt werden, indem man die Verbindung der Formel (X) 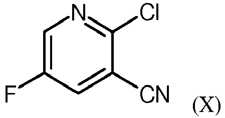 in einem inerten Lösungsmittel mit Hydrazinhydrat zur Verbindung der Formel (XI) 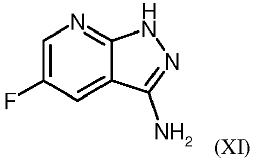 zyklisiert, diese anschliessend in einem inerten Lösungsmittel in Gegenwart einer geeigneten Lewis-Säure zunächst mit Isopentylnitrit zum entsprechenden Diazoniumsalz umsetzt, und dieses dann direkt mit Natriumiodid in die Verbindung der Formel (XII) 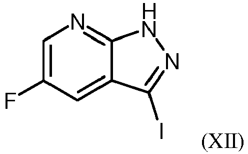 überführt, und diese im Folgenden in einem inerten Lösungsmittel in Gegenwart einer geeigneten Base mit der Verbindung der Formel (XIII) 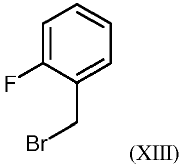 umsetzt.

Inerte Lösungsmittel für den Verfahrensschritt (X) → (XI) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist 1,2-Ethandiol.

Die Reaktion (X) → (XI) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XI) → (XII) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*.*N'*-Dimethylpropylenharnstoff (DMPU), *N*-Methylpyrrolidon (NMP), Pyridin oder Acetonitril. Bevorzugt ist DMF.

Als Lewis-Säuren für den Verfahrensschritt (XI) → (XII) eignen sich Bortrifluorid-Diethylether-Komplex, Cer(IV)ammoniumnitrat (CAN), Zinn(II)chlorid, Lithiumperchlorat, Zink(II)chlorid, Indium(III)chlorid oder Indium(III)bromid. Bevorzugt ist Bortrifluorid-Diethylether-Komplex.

Die Reaktion (XI) → (XII) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XII) + (XIII) → (II) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*.*N'*-Dimethylpropylenharnstoff (DMPU), *N*-Methylpyrrolidon (NMP), Pyridin, Acetonitril. Bevorzugt ist DMF.

Geeignete Basen für den Verfahrensschritt (XII) + (XIII) → (II) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Cäsiumcarbonat.

Die Reaktion (XII) + (XIII) → (II) wird im Allgemeinen in einem Temperaturbereich von 0°C bis +60°C, bevorzugt bei +10°C bis +25°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Das beschriebene Herstellverfahren kann durch das folgende Syntheseschema (Schema 4) beispielhaft verdeutlicht werden:

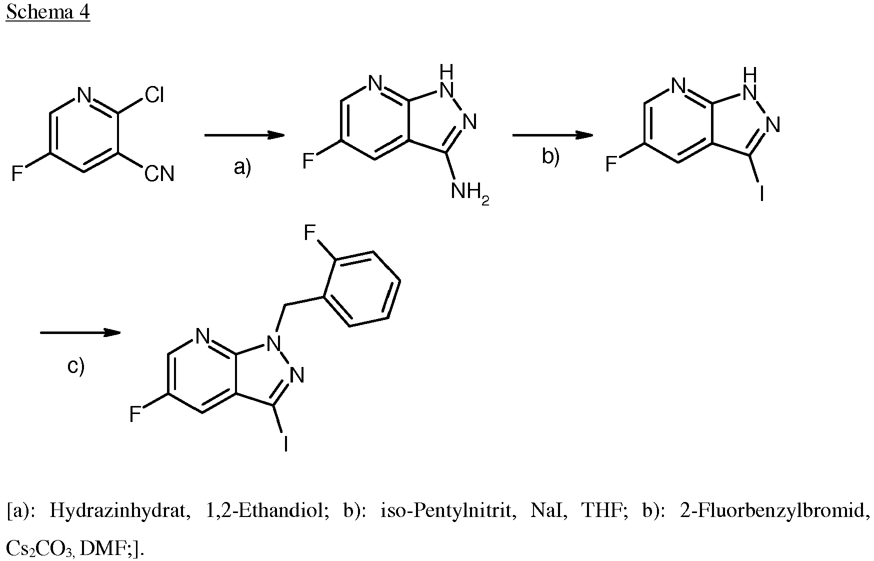

Weiterer Gegenstand der vorliegenden Erfindung ist die Verbindung:

### 5-Fluor-1-(2-fluorbenzyl)-3-iod-1H-pyrazolo[3,4-b]pyridin

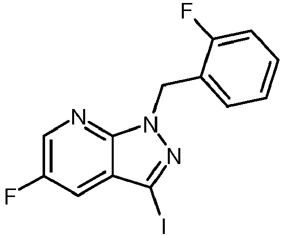

Weiterer Gegenstand der vorliegenden Erfindung ist die Verbindung:

### 5-Fluor-3-iod-1H-pyrazolo[3,4-b]pyridin

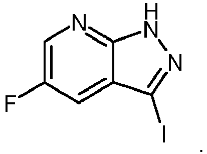

Die Verbindungen der Formeln (III) und (XIII) sind kommerziell erhältlich, literaturbekannt oder können in Analogie zu literaturbekannten Verfahren hergestellt werden.

Die Verbindung der Formel (X) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 5) hergestellt werden:

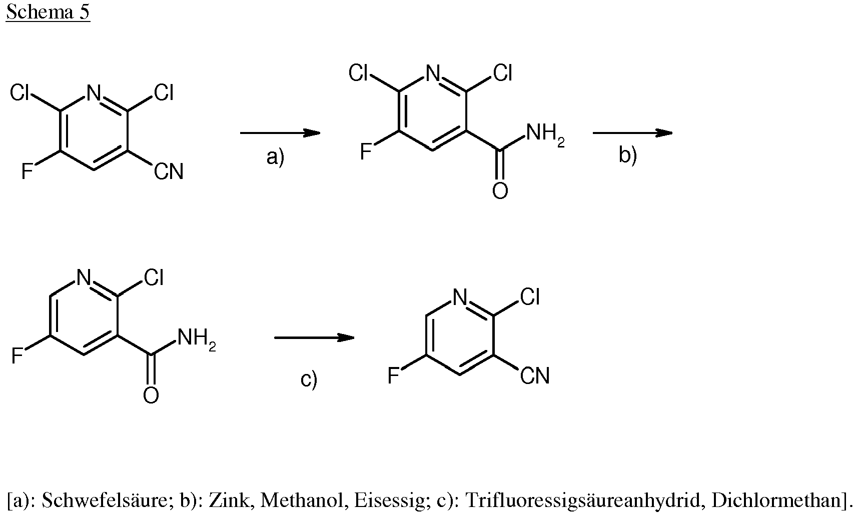

Die erfindungsgemäßen Verbindungen wirken als Stimulatoren der löslichen Guanylatcyclase und weisen ein gleiches oder verbessertes therapeutisches Profil gegenüber den aus dem Stand der Technik bekannten Verbindungen auf, wie beispielsweise hinsichtlich ihrer *in-vivo* Eigenschaften, wie beispielsweise ihrem pharmakokinetischem und pharmakodynamischem Verhalten und/oder ihrer Dosis-Wirkungsbeziehung und/oder ihres Sicherheitsprofils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junlctionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", alters assoziierten Lern- und Gedächtnisstörungen, alters assoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Anryolateralsklerose (ALS), Huntington'scher Krankheit, Denryelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunlctionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Offenbarungsgegenstand ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: berechnet
- br s: breites Singulett (bei NMR)
- DCI: direkte chemische Ionisation (bei MS)
- dec.: Zersetzungspunkt
- DMF: Dimethylformamid
- DMSO: Dimethylsulfoxid
- DSC: Dynamische Differenzkalorimetrie
- d. Th.: der Theorie (bei Ausbeute)
- eq.: Äquivalent(e)
- ESI: Elektrospray-Ionisation (bei MS)
- Et: Ethyl
- gef.: gefunden
- h: Stunde(n)
- HPLC: Hochdruck-, Hochleistungsflüssigchromatographie
- HRMS: hochaufgelöste Massenspektrometrie
- konz.: konzentriert
- LC-MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- PLM: Polararisations-Lichtmikroskop
- RT: Raumtemperatur
- Rₜ: Retentionszeit (bei HPLC)
- TGA: Thermogravimetrische Analyse
- THF: Tetrahydrofuran
- UV: Ultraviolett-Spektrometrie
- v/v: Volumen zu Volumen-Verhältnis (einer Lösung)

### LC/MS-Methoden:

**Methode 1:** Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

**Methode 2:** Instrument: Waters ACQUITY SQD UPLC System; Säule: Waters Acquity UPLC HSS T3 1.8 µ 50 x 1 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.

**Methode 3:** Instrument: Micromass Quattro Premier mit Waters UPLC Acquity; Säule: Thermo Hypersil GOLD 1.9 µ 50 x 1 mm; Eluent A: 1 1 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.

### Allgemeine Methoden:

**PLM:** Die Polarisations-Lichtmikroskopie wurde mit einem Clemex PS3 Polarisations-Lichtmikroskop Partikelgrössen-System mit einem Leica DM Mikroskop durchgeführt, das mit 50X, 100X, 200X, and 500X Linsen, einer hochauflösenden monochromen 1600x1200 Pixel Digitalkamera und einer motorisierten X-Y Marzhauser Station (gesteuert von einem Clemex ST-2000 Steuergerät), ausgestattet ist. Die Proben des kristallinen Materials wurden auf einem Glas-Objektträger (76x26 mm) in einem Tropfen Öl vermessen, wobei die Probe mit einem Deckglas (22x40 mm) bedeckt wurde.

**DSC:** Die dynamische Differenzkalorimetrie wurde zur Bestimmung der Schmelzpunkte verwendet. Die Bestimmung erfolgte mit einem Mettler-Toledo 823^{e} DSC-Gerät, das mit einem TSO801RO Probenroboter und der STAR^{e} Software ausgestattet ist. Es wurden ca. 1.5 bis 3 mg der Probe in eine kleine Aluminiumpfanne eingewogen, die Loch-Kappen verschlossen wurden. Der Wärmefluss wurde in einem Temperaturbereich von 30 bis 400°C bei einer Aufheizrate von 10°C/min und unter Argonfluss von 30 ml/min gemessen.

**TGA:** Die thermogravimetrische Analyse wurde mit einen Mettler-Toledo TGA/SDTA851^{e} TGA-Gerät durchgeführt, das mit einem TSO801RO Probenroboter und der STAR^{e} Software ausgestattet ist. Es wurden ca. 1.5 bis 3 mg der Probe in eine kleine offene Aluminiumpfanne (100µl) eingewogen. Das Gewicht der Probe wurde in einem Temperaturbereich von 30 bis 400°C bei einer Aufheizrate von 10°C/min und unter Argonfluss von 30 ml/min gemessen.

Die Elementaranalysen wurde nach den dem Fachmann bekannten Methoden gemäß Industrienorm DIN-ISO 17025 von Currenta GmbH & Co. durchgeführt.

**Ausgangsverbindungen und Intermediate:**

### Beispiel 1A

### 2,6-Dichlor-5-fluornicotinamid

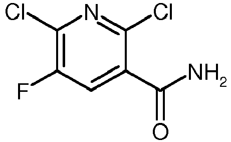

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.
Ausbeute: 24.5 g (90 % d. Theorie)
¹H-NMR (400 MHz, DMSO-d₆): δ = 7.95 (br s, 1H), 8.11 (br s, 1H), 8.24 (d, 1H).

### Beispiel 2A

### 2-Chlor-5-fluornicotinamid

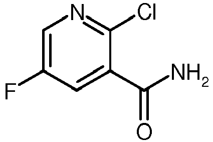

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min ausgerührt. Es wurde erneut abgesaugt und mit Diethylether nachgewaschen und trockengesaugt.
Ausbeute: 20.2 g (53 % d. Theorie)
¹H-NMR (400 MHz, DMSO-d₆): δ = 7.87 (br s, 1H), 7.99 (dd, 1H), 8.10 (br s, 1H), 8.52 (d, 1H).

### Beispiel 3A

### 2-Chlor-5-fluornicotinonitril

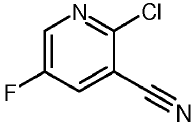

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.
Ausbeute: 42.1 g (90 % d. Theorie).
¹H-NMR (400 MHz, DMSO-d₆): δ = 8.66 (dd, 1H), 8.82 (d, 1H).

### Beispiel 4A

### 5-Fluor-1H-pyrazolo[3,4-b]pyridin-3-amin

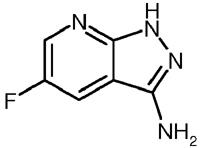

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml, 2.459 mol) versetzt. Es wurde unter Rühren für 4 h zum Rückfluss erhitzt. Beim Abkühlen fiel das Produkt aus. Die gelben Kristalle wurden mit Wasser (380 ml) versetzt und 10 min. bei RT ausgerührt. Anschliessend wurde die Suspension über eine Fritte abgesaugt, mit Wasser (200 ml) und mit -10°C-kaltem THF (200 ml) nachgewaschen. Der Rückstand wurde im Hochvakuum über Phosphorpentoxid getrocknet.
Ausbeute: 22.8 g (61 % d. Theorie)
¹H-NMR (400 MHz, DMSO-d₆): δ = 5.54 (s, 2H), 7.96 (dd, 1H), 8.38 (m, 1H), 12.07 (m, 1H).

### Beispiel 5A

### 5-Fluor-3-iod-1H-pyrazolo[3,4-b]pyridin

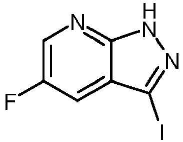

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.8 l) gegeben und zweimal mit Essigsäureethylester (je 487 ml) extrahiert. Die gesammelten organischen Phasen wurden mit gesättigter wässriger Natriumchlorid-Lösung (244 ml) gewaschen, getrocknet, filtriert und eingeengt. Man erhielt 12.1 g (86%-ige Reinheit, 60 % d. Th.) der gewünschten Verbindung als braunen Feststoff. Das Rohprodukt wurde ohne weitere Reinigung umgesetzt.
LC-MS (Methode 1): Rₜ = 1.68 min; MS (ESIpos): m//z = 264 (M+H)⁺

### Beispiel 6A

### 5-Fluor-1-(2-fluorbenzyl)-3-iod-1H-pyrazolo[3,4-b]pyridin

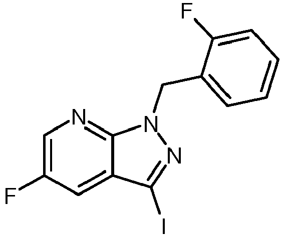

In DMF (2538 ml) wurden 141 g (462.11 mmol) der Verbindung aus Beispiel 5A vorgelegt und anschließend 96.09 g (508.32 mmol) 2-Fluorbenzylbromid sowie 165.62 g (508.32 mmol) Cäsiumcarbonat zugefügt. Die Mischung wurde zwei Stunden bei RT gerührt. Anschließend wurde die Reaktionsmischung auf gesättigte wässrige Natriumchlorid-Lösung (13670 ml) gegeben und zweimal mit Essigsäureethylester (5858 ml) extrahiert. Die gesammelten organischen Phasen wurden mit gesättigter wässriger Natriumchlorid-Lösung (3905 ml) gewaschen, getrocknet, filtriert und eingeengt. Der Rückstand wurde an Kieselgel (Laufmittel: Petrolether/Essigsäureethylester 97:3) chromatographiert und die Produktfraktionen eingeengt. Der erhaltene Feststoff wurde in Dichlormethan gelöst und einmal mit gesättigter wässriger Natriumthiosulfatlösung (500 ml) und anschliessend mit gesättigter wässriger Natriumchlorid-Lösung (500 ml) gewaschen. Es wurde zur Trockene eingeengt und der Rückstand mit Diethylether aufgeschlämmt, abgesaugt und am Hochvakuum getrocknet. Man erhielt 106.6 g (62 % d. Th.) der gewünschten Verbindung.
¹H-NMR (400 MHz, DMSO-d₆): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 7A

### 2-[5-Fluor-1-(2-fluorbenzyl)-1H-pyrazolo[3,4-b]pyridin-3-yl]-5-nitropyrimidin-4,6-diamin

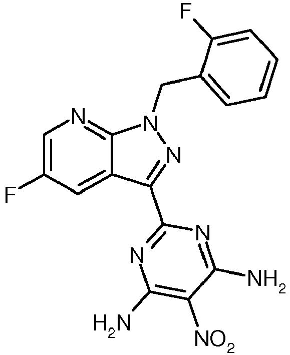

In 1,4-Dioxan (86 ml) wurden 860 mg (2.32 mmol) der Verbindung aus Beispiel 6A unter Argon vorgelegt und die Reaktionsmischung 10 min mit Argon gespült. Danach wurden 3.51 ml (6.95 mmol) Hexabutyldizinn sowie 483 mg (2.55 mmol) 2-Chlor-5-nitropyrimidin-4,6-diamin (Herstellung erfolgte analog zu: Helvetica Chimica Acta (1951), 34, 835-40) zugefügt. Anschließend wurden 860 mg (0.744 mmol) Tetrakis(triphenylphosphin)-palladium(0) zugegeben und die Reaktionsmischung über Nacht zum Rückfluss erhitzt. Danach wurde auf RT abgekühlt, mit Wasser versetzt und zweimal mit Essigsäureethylester extrahiert. Die gesammelten organischen Phasen wurden über Natriumsulfat getrocknet, filtriert und eingeengt. Der Rückstand wurde in Essigsäureethylester ausgerührt, der Feststoff abfiltriert und im Hochvakuum getrocknet. Man erhielt 355 mg (62%-ige Reinheit, 24% d. Th.) der gewünschten Verbindung. Das Rohprodukt wurde ohne weitere Reinigung umgesetzt.
LC-MS (Methode 2): Rₜ = 1.03 min
MS (ESIpos): m//z = 399 (M+H)⁺

### Beispiel 8A

### 5-Fluor-1-(2-fluorbenzyl)-1H-pyrazolo[3,4-b]pyridin-3-carbonitril

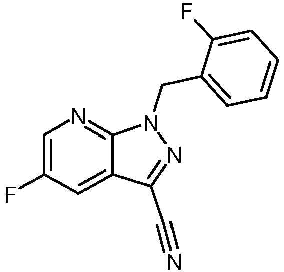

Eine Suspension aus 16.03 g (43.19 mmol) 5-Fluor-1-(2-fluorbenzyl)-3-iod-1H-pyrazolo[3,4-b]pyridin (Beispiel 6A) und 4.25 g (47.51 mmol) Kupfercyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, getrocknet und im Vakuum eingeengt. Das Rohprodukt wurde ohne weitere Reinigung umgesetzt.
Ausbeute: 11.1 g (91 % d. Theorie)
¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 9A

### 5-Fluor-1-(2-fluorbenzyl)-1H-pyrazolo[3,4-b]pyridin-3-carboximidamid-Acetat

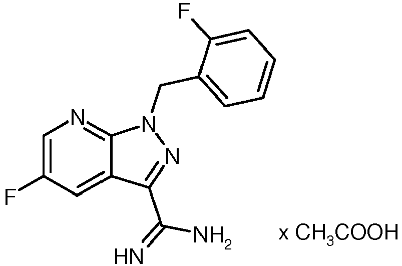

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 8A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde bis zur Trockene eingeengt und der Rückstand in Wasser (100 ml) und Essigsäureethylester (100 ml) aufgenommen und mit 2N Natronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wurde über Phosphorpentoxid im Hochvakuum getrocknet.
Ausbeute: 9.6 g (78 % d. Th.)
MS (ESIpos): m//z = 288 (M+H)⁺
¹H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Beispiel 10A

### 2-[5-Fluor-1-(2-fluorbenzyl)-1H-pyrazolo[3,4-b]pyridin-3-yl]-5-[(E)-phenyldiazenyl]pyrimidin-4,6-diamin

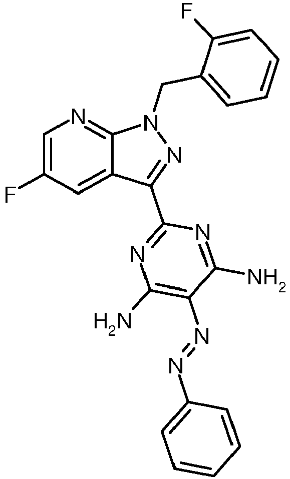

Zu Wasser (40 ml) und konz. Salzsäure (7.07 ml) wurden unter Rühren 3.85 g (41.34 mmol) Anilin gegeben und auf 0°C gekühlt. Anschliessend wurde eine Lösung von 2.85 g (41.34 mmol) Natriumnitrit in Wasser (21 ml) zwischen 0°C und 5°C zugetropft und 15 min bei 0°C nachgerührt. Danach wurde bei 0°C eine Lösung aus 4.28 g (52.25 mmol) Natriumacetat in Wasser (19 ml) zügig zugetropft und dann unter gutem Rühren eine Lösung aus 2.73 g (41.34 mmol) Malonsäuredinitril in Ethanol (10 ml) zugetropft. Nach 2 h bei 0°C wurde der entstandene Niederschlag abgesaugt und dreimal mit Wasser (je 50 ml) und mit Petrolether (50 ml) gewaschen. Der noch feuchte Rückstand wurde in DMF (46 ml) gelöst und in eine Lösung aus 9.5 g (33.07 mmol) 5-Fluor-l-(2-fluorbenzyl)-1H-pyrazolo[3,4-b]pyridin-3-carboximidamid-Acetat (Beispiel 9A) in DMF (46 ml) und Triethylamin (5.76 ml) bei genau 85°C zugetropft. Anschließend wurde 4 h bei 100°C nachgerührt und über Nacht auf RT abkühlen lassen. Die Mischung wurde auf Wasser (480 ml) gegossen und 1 h bei RT ausgerührt. Nach Absaugen des Niederschlags wurde dieser zweimal mit Wasser (je 100 ml) und zweimal mit Methanol (je 50 ml) nachgewaschen, und anschliessend im Hochvakuum getrocknet.
Ausbeute: 9.6 g (59 % d. Th.)
LC-MS (Methode 2): Rₜ = 1.21 min
MS (ESIpos): m/z = 458 (M+H)⁺

### Beispiel 11A

### 2-[5-Fluor-1-(2-fluorbenzyl)-1H-pyrazolo[3,4-b]pyridin-3-yl]pyrimidin-4,5,6-triamin

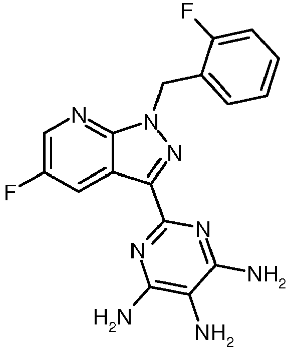

### Variante A: Darstellung ausgehend von Beispiel 7A:

In Pyridin (30 mL) wurden 378 mg (0.949 mmol) der Verbindung aus Beispiel 7A vorgelegt und anschließend 143 mg (0.135 mmol) Palladium (10%ig auf Kohle) zugefügt. Die Mischung wurde über Nacht bei RT unter Wasserstoff-Normaldruck hydriert. Anschließend wurde die Suspension durch Kieselgur filtriert und der Filterkuchen mit Ethanol nachgewaschen. Das Filtrat wurde eingeengt und lieferte 233 mg (81%ig, 51% d. Th.) der gewünschten Verbindung, die ohne weitere Reinigung umgesetzt wurden.

### Variante B: Darstellung ausgehend von Beispiel 10A:

In DMF (800 ml) wurden 39.23 g (85.75 mmol) der Verbindung aus Beispiel 10A vorgelegt und anschliessend 4 g Palladium (10%ig auf Kohle) zugefügt. Es wurde unter Rühren über Nacht bei Wasserstoff-Normaldruck hydriert. Der Ansatz wurde über Kieselgur filtriert und mit wenig DMF und danach mit wenig Methanol nachgewaschen und zur Trockene eingeengt. Der Rückstand wurde mit Essigsäureethylester versetzt und kräftig gerührt, der Niederschlag abgesaugt, mit Essigsäureethylester und Diisopropylether nachgewaschen und über Sicapent im Hochvakuum getrocknet.
Ausbeute: 31.7 g (100 % d. Theorie)
LC-MS (Methode 2): Rₜ = 0.78 min
MS (ESIpos): m/z = 369 (M+H)⁺

### Ausführungsbeispiele:

### Beispiel 1

### Methyl-{4,6-diamino-2-[5-fluor-1-(2-fluorbenzyl)-1H-pyrazolo[3,4-b]pyridin-3-yl]pyrimidin-5-yl}carbamat

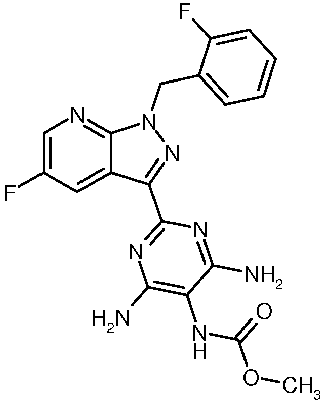

In Pyridin (600 ml) wurden 31.75 g (86.20 mmol) der Verbindung aus Beispiel 11A unter Argon vorgelegt und auf 0°C abgekühlt. Anschließend wurde eine Lösung von 6.66 ml (86.20 mmol) Chlorameisensäuremethylester in Dichlormethan (10 ml) zugetropft und die Mischung 1h bei 0°C gerührt. Danach wurde die Reaktionsmischung auf RT gebracht, im Vakuum eingeengt und mehrfach mit Toluol kodestilliert. Der Rückstand wurde mit Wasser/Ethanol verrührt und danach über eine Fritte abgesaugt und nachfolgend mit Ethanol und Essigsäureethylester gewaschen. Anschliessend wurde der Rückstand abermals mit Diethylether verrührt, abgesaugt und anschliessend im Hochvakuum getrocknet.
Ausbeute: 24.24 g (65 % d. Theorie)
LC-MS (Methode 2): Rₜ = 0.79 min
MS (ESIpos): m/z = 427 (M+H)⁺
¹H-NMR (400 MHz, DMSO-d₆): δ = 3.62 (br s, 3H), 5.79 (s, 2H), 6.22 (br s, 4H), 7.10 - 7.19 (m, 2H), 7.19 - 7.26 (m, 1H), 7.32 - 7.40 (m, 1H), 7.67 und 7.99 (2 br s, 1H), 8.66 (m, 1H), 8.89 (dd, 1H).

### Beispiel 2

### Methyl-{4,6-diamino-2-[5-fluor-1-(2-fluorbenzyl)-1H-pyrazolo[3,4-b]pyridin-3-yl]pyrimidin-5-yl}methylcarbamat

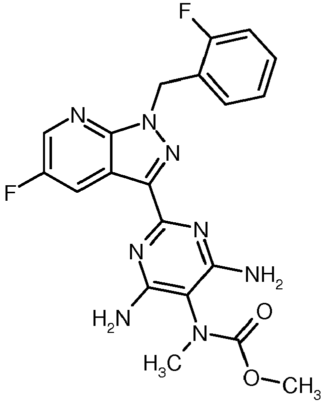

200 mg (0.469 mmol) Methyl-{4,6-diamino-2-[5-fluor-1-(2-fluorbenzyl)-1H-pyrazolo[3,4-b]pyridin-3-yl]pyrimidin-5-yl}carbamat (Beispiel 1) wurden in THF (5ml) bei 0°C vorgelegt. Danach wurden 0.704 ml (0.704 mmol) Lithiumhexamethyldisilazan-Lösung (IM in THF) zugegeben und 20 min bei dieser Temperatur gerührt. Anschliessend wurden 43.8 µl (0.704 mmol) Iodmethan zugegeben und auf RT erwärmt. Nach 1h bei dieser Temperatur wurde mit Wasser abgebrochen (1 ml), eingeengt und der Rückstand mittels präparativer RP-HPLC (Wasser (+0.05 % Ameisensäure)-Acetonitril Gradient) getrennt.
Ausbeute: 90 mg (44 % d. Theorie)
LC-MS (Methode 2): Rₜ = 0.85 min
MS (ESIpos): m/z = 441 (M+H)⁺
¹H-NMR (400 MHz, DMSO-d₆): δ = 3.00 (s, 3H), 3.53 und 3.66 (2 s, 3H), 5.81 (s, 2H), 6.57 (br s, 4H), 7.13 (m, 2H), 7.22 (m, 1H), 7.35 (m, 1H), 8.67 (m, 1H), 8.87 (dd, 1H).

### Beispiel 3

### Methyl-{4,6-diamino-2-[5-fluor-1-(2-fluorbenzyl)-1H-pyrazolo[3,4-b]pyridin-3-yl]pyrimidin-5-yl}(2,2,2-trifluorethyl)carbamat

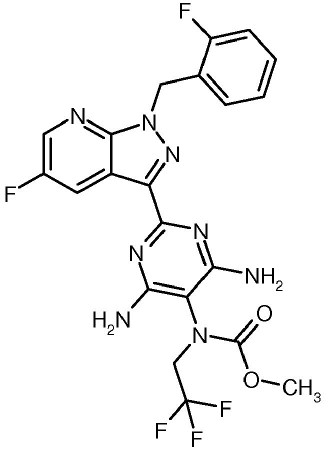

Es wurden 3.470 g (8.138 mmol) der Verbindung aus Beispiel 1 in 35 ml THF suspendiert, bei 0°C mit 358 mg (8.952 mmol) Natriumhydrid (60%-ige Suspension in Mineralöl) versetzt und 90 min bei 0°C gerührt, wobei sich eine Lösung bildete. Es wurden 2.519 g (8.952 mmol) 2,2,2-Trifluorethyltrichlormethansulfonat addiert und das Gemisch für 48 h bei RT gerührt. Anschliessend wurde mit Wasser verrührt und am Rotationsverdampfer eingeengt. Der Rückstand wurde in Essigsäureethylester aufgenommen, die organische Phase zweimal mit Wasser gewaschen und über Natriumsulfat getrocknet. Es wurden 5.005 g der Zielverbindung erhalten (79 % d. Th., Reinheit nach HPLC 65%). 250 mg des Rückstands wurden mittels präparativer HPLC gereinigt (Eluent: Methanol/Wasser, Gradient 30:70 → 90:10).
LC-MS (Methode 2): Rₜ = 0.97 min; MS (EIpos): m/z = 509 [M+H]⁺.
¹H-NMR (400 MHz, DMSO-d₆): δ [ppm] = 3.63 (s, 3H), 4.06-4.15 (m, 2H), 5.80 (s, 2H), 6.46 (br s, 4H) 7.11-7.15 (m, 2H), 7.20-7.25 (m, 1H), 7.33-7.38 (m, 1H), 8.66 (dd, 1H), 8.91 (dd, 1H).

### Beispiel 4

### Methyl-{4,6-diamino-2-[5-fluor-1-(2-fluorbenzyl)-1H-pyrazolo[3,4-b]pyridin-3-yl]pyrimidin-5-yl}carbamat-Hydrochlorid

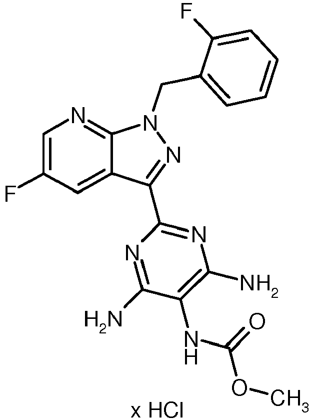

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL Isopropanol und 235 µL (0.235 mmol) 1M Salzsäure gegeben, und die Lösung wurde bei RT gerührt bis die Lösungsmittel verdampft waren. Nach Trocknung an Luft wurden 102 mg (94% d. Th.) der Titelverbindung isoliert.
PLM(100x): kristallin
DSC: 224°C (dec., ΔH = 189 J/g)
TGA: 1% Gewichtsverlust bei 80°C
LC-MS (Methode 3): Rₜ = 0.91 min
MS (ESIpos): m/z = 427 (M+H)⁺
¹H-NMR (400 MHz, DMSO-d₆): δ [ppm] = 3.35 und 3.65 (2 s, 3H), 5.92 (s, 2H), 7.15 (dd, 1H), 7.25 (m, 2H), 7.37 (m, 1H), 7.75 (br s, 4H), 8.08 und 8.39 (2 s, 1H), 8.82 (m, 2H), 13.2 (br s, 1H).
Elementaranalyse für C₁₉H₁₆F₂N₈O₂ + HCl:
berechnet: %C 49.31; %H 3.70; %N 24.21;
gemessen: %C 49.5; %H 3.7; %N 24.3.

### Beispiel 5

### Methyl-{4,6-diamino-2-[5-fluor-1-(2-fluorbenzyl)-1H-pyrazolo[3,4-b]pyridin-3-yl]pyrimidin-5-yl}carbamat-Sulfat

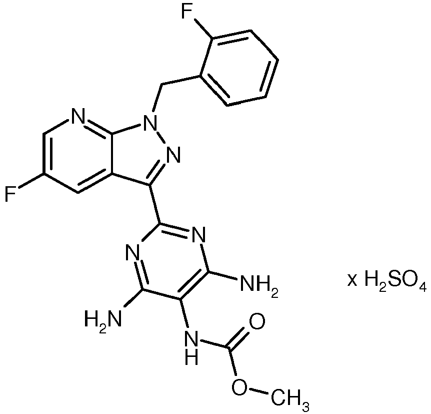

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL Isopropanol und eine Lösung von 938 µL (0.235 mmol) 0.25M Schwefelsäure gegeben, und die Lösung wurde bei RT gerührt bis die Lösungsmittel verdampft waren. Nach Trocknung an Luft wurden 103 mg (83.7% d. Th.) der Titelverbindung isoliert.
PLM (100x): kristallin
DSC: 242°C (dec., ΔH = 115 J/g)
TGA: kein Gewichtsverlust vor dem Zersetzungspunkt
LC-MS (Methode 3): Rₜ = 0.91 min
MS (ESIpos): m/z = 427 (M+H)⁺
¹H-NMR (400 MHz, DMSO-d₆): δ [ppm] = 3.56 und 3.66 (2 s, 3H), 5.93 (s, 2H), 7.16 (m, 2H), 7.25 (dd, 1H), 7.38 (m, 1H), 7.59 (br s, 4H), 8.03 und 8.32 (2 s, 1H), 8.82 (m, 2H), 13.0 (br s, 1H).
Elementaranalyse für C₁₉H₁₆F₂N₈O₂ + H₂SO₄:
berechnet: %C 43.51; %H 3.46; %N 21.37;
gemessen: %C 43.6; %H 3.4; %N 21.2.

### Beispiel 6

### Methyl-{4,6-diamino-2-[5-fluor-1-(2-fluorbenzyl)-1H-pyrazolo[3,4-b]pyridin-3-yl]pyrimidin-5-yl}carbamat-Phosphat

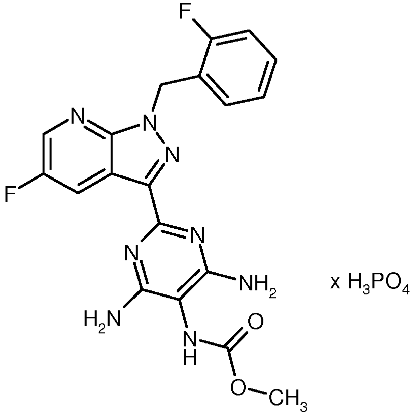

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL THF und eine Lösung von 16 µL (0.235 mmol) 85%-iger Phosphorsäure in 0.3 mL Wasser gegeben, und die Lösung wurde bei RT gerührt bis die Lösungsmittel verdampft waren. Nach Trocknung an Luft wurden 105 mg (85.4% d. Th.) der Titelverbindung isoliert.
PLM (100x): kristallin
DSC: 183°C (dec., ΔH = 65 J/g)
TGA: 6% Gewichtsverlust vor dem Zersetzungspunkt
LC-MS (Methode 3): Rₜ = 0.91 min
MS (ESIpos): m/z = 427 (M+H)⁺
¹H-NMR (400 MHz, DMSO-d₆): δ [ppm] = 3.57 und 3.62 (2 s, Wasser-Signal überlagert Methyl-Signal, 3H), 5.79 (s, 2H), 6.22 (br s, 4H), 7.15 (m, 2H), 7.22 (dd, 1H), 7.36 (m, 1H), 7.67 und 7.99 (2 s, 1H), 8.66 (m, 1H), 8.90 (m, 1H).
³¹P-NMR (400 MHz, DMSO-d₆): δ [ppm] = -1.1
Elementaranalyse für C₁₉H₁₆F₂N₈O₂ + H₃PO₄ + 2 H₂O:
berechnet: %C 40.72; %H 4.14; %N 19.99;
gemessen: %C 40.5; %H 4.0; %N 19.5.

### Beispiel 7

### Methyl-{4,6-diamino-2-[5-fluor-1-(2-fluorbenzyl)-1H-pyrazolo[3,4-b]pyridin-3-yl]pyrimidin-5-yl}carbamat-Mesylat

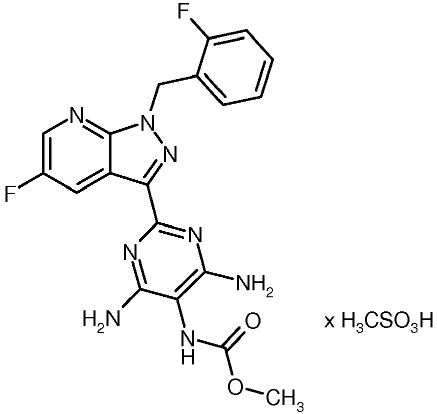

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL Ethanol und eine Lösung von 22.5 mg (0.235 mmol) Methansulfonsäure in 0.3 ml Wasser gegeben, und die Lösung wurde bei RT gerührt bis die Lösungsmittel verdampft waren. Nach Trocknung an Luft wurden 103 mg (84% d. Th.) der Titelverbindung isoliert.
PLM (100x): kristallin
DSC: 154°C (ΔH = 11.7 J/g), 167°C (ΔH = -5 J/g), 215.2°C (dec, ΔH = 56.1 J/g)
TGA: gradueller Gewichtsverlust während der Messung
LC-MS (Methode 3): Rₜ = 0.91 min
MS (ESIpos): m/z = 427 (M+H)⁺
¹H-NMR (400 MHz, DMSO-d₆): δ [ppm] = 2.31 (s, 3H), 3.57 und 3.66 (2 s, 3H), 5.93 (s, 2H), 7.17 (m, 2H), 7.25 (dd, 1H), 7.39 (m, 1H), 7.66 (s br, 4H), 8.06 und 8.34 (2 s, 1H), 8.81 (dd, 1H), 8.83 (s, 1H), 13.0 (br s, 1H).
Elementaranalyse für C₁₉H₁₆F₂N₈O₂ + CH₄O₃S + H₂O:
berechnet: %C 44.44; %H 4.10; %N 20.7;
gemessen: %C 44.3; %H 4.1; %N 20.2.

### Beispiel 8

### Methyl-{4,6-diamino-2-[5-fluor-1-(2-fluorbenzyl)-1H-pyrazolo[3,4-b]pyridin-3-yl]pyrimidin-5-yl }carbamat-Ethan-1,2-disulfonat

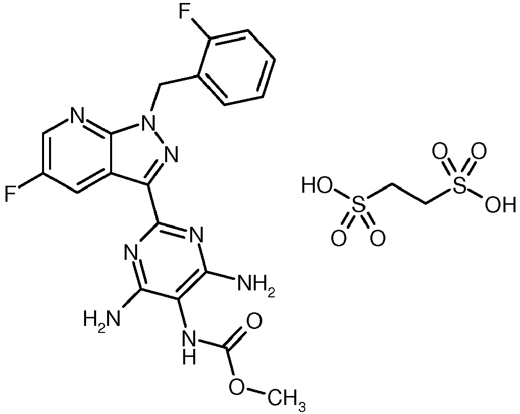

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL Isopropanol und 44.6 mg (0.235 mmol) Ethan-1,2-disulfonsäure gegeben, und die Lösung wurde bei RT gerührt bis die Lösungsmittel verdampft waren. Nach Trocknung an Luft wurden 111 mg (73.7% d. Th.) der Titelverbindung isoliert.
PLM (100x): überwiegend kristallin
DSC: 97°C (dec., ΔH = 103 J/g)
TGA: gradueller Gewichtsverlust während der Messung
LC-MS (Methode 3): Rₜ = 0.90 min
MS (ESIpos): m/z = 427 (M+H)⁺
¹H-NMR (400 MHz, DMSO-d₆): δ [ppm] = 2.66 (s, 4H), 3.57 und 3.66 (2 s, Wasser-Signal überlappt Methyl-Signal, 3H), 5.93 (s, 2H), 7.17 (m, 2H), 7.25 (m, 1H), 7.39 (m, 1H), 8.05 und 8.35 (2 s, 1H), 8.80 (dd, 1H), 8.84 (s, 1H).
Elementaranalyse für C₁₉H₁₆F₂N₈O₂ + C₂H₆O₆S₂ + 0.25 H₂O + 0.25 C₄H₈O₂:
berechnet: %C 41.09; %H 3.84; %N 17.42;
gemessen: %C 41.2; %H 4.2; %N 17.6.

### Beispiel 9

### Methyl-{4,6-diamino-2-[5-fluor-1-(2-fluorbenzyl)-1H-pyrazolo[3,4-b]pyridin-3-yl]pyrimidin-5-yl}carbamat-Maleat

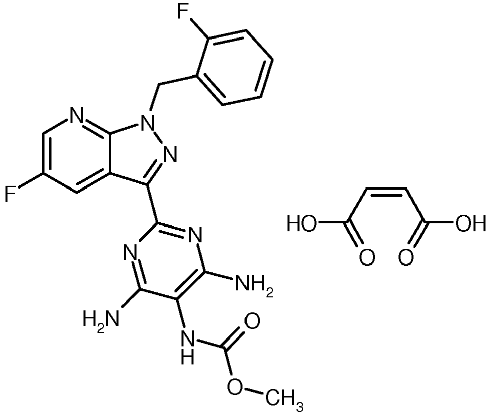

Eine Lösung von 100 mg (0.235 mmol) Beispiel 1 in 2 mL 1,4-Dioxan wurde in einem braunen 5 mL Glasfläschchen hergestellt. Zu dieser Lösung wurden nacheinander 2 mL Isopropanol und 27.2 mg (0.235 mmol) Maleinsäure gegeben, und die Lösung wurde bei RT gerührt bis die Lösungsmittel verdampft waren. Nach Trocknung an Luft wurden 108 mg (84.9% d. Th.) der Titelverbindung isoliert.
PLM (100x): kristallin
DSC: 192°C (dec, ΔH = 173 J/g)
TGA: 3% Gewichtsverlust vor dem Zersetzungspunkt
LC-MS (Methode 3): Rₜ = 0.91 min
MS (ESIpos): m/z = 427 (M+H)⁺
¹H-NMR (400 MHz, DMSO-d₆): δ [ppm] = 3.56 und 3.64 (2 s, überlappt vom Dioxan-Signal, 3H), 5.85 (s, 2H), 6.16 (s, 2H), 6.9 (br s, 4H), 7.15 (m, 2H), 7.23 (dd, 1H), 7.37 (m, 1H), 7.85 und 8.13 (2 s, 1H), 8.73 (s, 1H), 8.86 (dd, 1H).
Elementaranalyse für C₁₉H₁₆F₂N₈O₂ + C₄H₄O₄ + 0.5 H₂O + 0.5 C₄H₈O₂:
berechnet: %C 50.42; %H 4.23; %N 18.82;
gemessen: %C 50.7; %H 3.9 ; %N 18.8.

### Beispiel 10

### Methyl-{4,6-diamino-2-[5-fluor-1-(2-fluorbenzyl)-1H-pyrazolo[3,4-b]pyridin-3-yl]pyrimidin-5-yl}carbamat-Nitrat

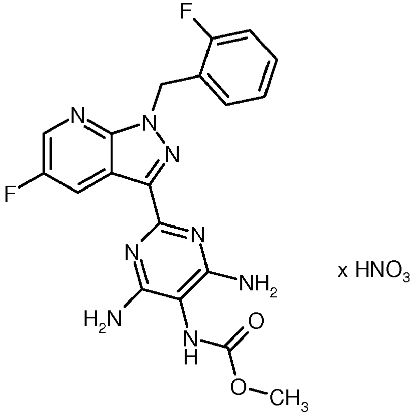

Eine Lösung von 100 mg (0.235 mmol) Beispiel 1 in 2 mL Isopropanol wurde in einem braunen 5 mL Glasfläschchen hergestellt. Zu dieser Lösung wurden nacheinander 2 mL Isopropanol und 0.235 µL (0.235 mmol) 1M Salpetersäure gegeben, und die Lösung wurde bei RT gerührt bis die Lösungsmittel verdampft waren. Nach Trocknung an Luft wurden 103 mg (89.7% d. Th.) der Titelverbindung isoliert.
PLM (100x): kristallin
DSC: 175°C (dec, ΔH = -224 J/g)
TGA: 3% Gewichtsverlust vor dem Zersetzungspunkt
LC-MS (Methode 3): Rₜ = 0.91 min
MS (ESIpos): m/z = 427 (M+H)⁺
¹H-NMR (400 MHz, DMSO-d₆): δ [ppm] = 3.57 und 3.66 (2 s, 3H), 5.93 (s, 2H), 7.16 (m, 2H), 7.25 (dd, 1H), 7.38 (m, 1H), 7.65 (br s, 4H), 8.02 und 8.32 (2 s, 1H), 8.80 (dd, 1H), 8.83 (s, 1H), 13.0 (br s, 1H).
Elementaranalyse für C₁₉H₁₆F₂N₈O₂ + HNO₃ + 0.75 H₂O:
berechnet: %C 45.38; %H 3.71; %N 25.07;
gemessen: %C 45.4; %H 3.7; %N 25.0.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 1 | 958 |
| 2 | 251 |
| 3 | 515 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.3 |
| 2 | 0.1 |
| 3 | 0.03 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

Repräsentative Werte für die erfindungsgemäßen Verbindungen sind in der nachstehenden Tabelle (Tabelle 3) wiedergegeben:

**Tabelle 3:**

| **Beispiel 1** | | | | **Beispiel 2** | | |
|---|---|---|---|---|---|---|
| | **Vehikel** | **Dosis: 0.3 mg/kg p.o.** | **Dosis: 3.0 mg/kg p.o.** | | **Vehikel** | **Dosis: 0.3 mg/kg p.o.** |
| Stunden nach Substanzgabe | mittlerer Blutdruck (mmHg) | mittlerer Blutdruck (mmHg) | mittlerer Blutdruck (mmHg) | Stunden nach Substanzgabe | mittlerer Blutdruck (mmHg) | mittlerer Blutdruck (mmHg) |
| 0 | 153,6 | 151,0 | 149,0 | 0 | 149,0 | 161,3 |
| 1 | 164,5 | 148,4 | 129,3 | 1 | 158,2 | 145,7 |
| 2 | 146,7 | 136,4 | 111,1 | 2 | 142,2 | 130,5 |
| 3 | 145,4 | 130,6 | 106,0 | 3 | 149,2 | 121,5 |
| 4 | 149,6 | 129,1 | 107,8 | 4 | 152,3 | 123,1 |
| 5 | 149,9 | 132,8 | 109,3 | 5 | 155,8 | 121,6 |
| 6 | 151,6 | 125,6 | 106,8 | 6 | 147,3 | 123,8 |
| 7 | 147,6 | 131,9 | 110,9 | 7 | 147,3 | 124,4 |
| 8 | 147,5 | 131,8 | 109,8 | 8 | 149,3 | 128,7 |
| 9 | 150,8 | 138,5 | 114,3 | 9 | 151,0 | 133,7 |
| 10 | 149,8 | 138,3 | 114,5 | 10 | 152,5 | 139,2 |
| 11 | 154,0 | 138,9 | 115,6 | 11 | 150,3 | 137,9 |
| 12 | 145,3 | 137,7 | 118,8 | 12 | 146,2 | 143,0 |
| 13 | 141,1 | 142,9 | 120,4 | 13 | 143,2 | 146,0 |
| 14 | 147,8 | 144,5 | 122,8 | 14 | 146,4 | 149,2 |
| 15 | 151,0 | 143,8 | 125,8 | 15 | 150,5 | 152,3 |
| 16 | 151,3 | 146,3 | 131,5 | 16 | 145,3 | 155,5 |
| 17 | 148,8 | 141,8 | 124,7 | 17 | 143,9 | 156,3 |
| 18 | 149,2 | 138,4 | 129,6 | 18 | 150,3 | 157,3 |
| 19 | 151,2 | 149,2 | 135,6 | 19 | 147,7 | 156,9 |
| 20 | 152,6 | 145,1 | 135,2 | 20 | 153,4 | 156,3 |
| 21 | 146,3 | 142,1 | 129,3 | 21 | 148,6 | 149,3 |
| 22 | 146,3 | 141,8 | 128,3 | 22 | 153,3 | 147,1 |
| 23 | 150,3 | 143,6 | 130,2 | 23 | 151,1 | 153,1 |
| 24 | 147,4 | 135,1 | 130,8 | 24 | 154,1 | 152,3 |

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der Substanz werden in männlichen CD-1-Mäusen, männlichen Wister-Ratte und weiblichen Beagle-Hunden bestimmt. Das Applikationsvolumen beträgt bei Mäusen 10 mL/kg, bei Ratten 5 mL/kg und bei Hunden 0.5 mL/kg. Die intravenöse Gabe erfolgt bei Mäusen und Ratten mittels einer speziesspezifischen Plasma/DMSO-Formulierung (99/1) und bei Hunden mittels Wasser/PEG400/Ethanol (50/40/10). Den Ratten wird zur vereinfachten Blutabnahme vor der Substanzgabe ein Silikonkatheter in die rechte *Vena jugularis externa* gelegt. Die Operation erfolgt einen Tag vor dem Versuch unter Isofluran-Narkose und unter Gabe eines Analgetikums (Atropin/Rimadyl (3/1) 0.1 mL s.c.). Die Substanzgabe wird bei Mäusen i.v. bolus, bei Ratten i.v. bolus oder mittels 15-minütiger Infusion und Hunden mittels 15-minütiger Infusion durchgeführt. Die Blutabnahme erfolgt bei Mäusen nach 0.033, 0.083, 0.17, 0.5, 1, 2, 3, 4, 6, 7 und 24 Stunden sowie bei Hunden und Ratten bei 15-minütiger Infusion nach 0.083, 0.25, 0.28 0.33, 0.42, 0.75, 1, 2, 3, 4, 6, 7 und 24 Stunden sowie bei Ratten nach i.v. bolus-Applikation nach 0.033, 0.083, 0.17, 0.5, 1, 2, 3, 4, 6, 7 und 24 Stunden. Die orale Gabe der gelösten Substanz mittels Schlundsonde wird in allen Spezies basierend auf einer Wasser/PEG400/Ethanol-Formulierung (50/40/10) durchgeführt. Die Blutabnahme in Ratten und Hunden erfolgt hier nach 0.083, 0.17, 0.5, 0.75, 1, 2, 3, 4, 6, 7 und 24 Stunden. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den unbekannten Proben, Kalibrierproben und QC's wird ein interner Standard zugesetzt (ZK 228859) und es folgt eine Proteinfällung mittels Acetonitril im Überschuss. Nach Zugabe eines Ammoniumacetat-Puffers (0.01 M, pH 6.8 (Beispiel 1/3) oder pH 3.0 (Beispiel 2)) und folgendem Vortexen wird bei 1000 g zentrifugiert und der Überstand mittels LC-MS/MS (API 4000, AB Sciex) vermessen. Die chromatographische Trennung wird auf einer 1100-HPLC von Agilent durchgeführt. Das Injektionsvolumen beträgt 10 µL. Als Trennsäule wird eine auf 40°C temperierte Luna 5µ C8(2) 100A 50x2mm der Fa. Phenomenex verwendet. Es wird ein binärer Laufmittelgradient bei 400 µL/Min für Beispiel 1 verwendet (A: 0.01M Ammoniumacetat-Puffer pH 6.8, B: 0.1 % Ameisensäure in Acetonitril): 0 Min. (90 % A), 1 Min. (90 % A), 3.5 Min. (15 % A), 4.5 Min. (15 % A), 4.6 Min. (90 % A), 7 Min. (90 % A). Abweichend davon wird für Beispiel 2 ein binärer Laufmittelgradient bei 500 µL/Min verwendet (A: 0.01M Ammoniumacetat-Puffer pH 3.0, B: 0.1 % Ameisensäure in Acetonitril): 0 Min. (90 % A), 1.5 Min. (90 % A), 3.5 Min. (10 % A), 4.5 Min (10 % A), 5 Min. (90 % A), 7 Min (90 % A). Abweichend davon wird für Beispiel 3 folgender binärer Laufmittelgradient bei 500 µL/Min verwendet (A: 0.01M Ammoniumacetat-Puffer pH 6.8, B: 0.1 % Ameisensäure in Acetonitril): 0 Min. (90 % A), 1 Min. (90 % A), 3 Min. (10 % A), 4 Min. (10 % A), 4.5 Min. (90 % A), 6 Min. (90 % A). Die Temperatur der Turbo V Ionenquelle beträgt 500°C. Folgende MS-Geräteparameter werden benutzt: Curtain Gas 20 units (Beispiel 1), 16 units (Beispiel 2) beziehungsweise 15 units (Beispiel 3), Ionsprayvoltage 5 kV (Beispiel 1/2) oder 4.5 units (Beispiel 3), Gas 1 35 units (Beispiel 1/3) beziehungsweise 25 units (Beispiel 2), Gas 2 30 units, CAD Gas 4 units (Beispiel 1/3) oder 3 units (Beispiel 2). Die Quantifizierung der Substanzen erfolgt anhand der Peakhöhen oder -flächen aus extrahierten Ionenchromatogrammen spezifischer MRM-Experimente.

Aus den ermittelten Plasmakonzentration-Zeit-Verläufen werden die pharmakokinetischen Kenngrößen wie AUC, Cₘₐₓ, MRT (Mean residence time), t_{1/2} (Halbwertszeit) und CL (Clearance) mittels des validierten pharmakokinetischen Rechenprogramms KinEx (Vers. 2.5 und 3) berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 Min. im Taumelrollenmischer inkubiert. Nach Zentrifugation bei 1000g wird die Konzentration im Plasma gemessen (s.o.) und durch Quotientenbildung der c_{b}/cₚ-Wert ermittelt.

Nach intravenöser Gabe von 0.3 mg/kg der erfindungsgemäßen Verbindungen in Ratten wurden folgende Werte erhoben:

| Beispiel | 1* | 2** | 3** |
|---|---|---|---|
| AUC norm [kg·h/L] | 4.36 | 1.79 | 1.36 |
| CL_{Blut} [L/h/kg] | 0.29 | 0.53 | 1.02 |
| MRT [h] | 4.1 | 2.3 | 2.3 |
| t_{1/2} [h] | 3.4 | 1.7 | 1.9 |

| | | | |
|---|---|---|---|
| * 15-minütige Infusion ** i.v. bolus-Gabe | | | |

### B-5. Sicherheitsprofil

Die erfindungsgemäßen Substanzen zeigen ein überraschend günstiges Sicherheitsprofil in vivo, welches durch nichtklinische Sicherheitsstudien gemäß OECD (OECD guidelines for testing of chemicals, No. 407) und ICH (3BS2A) Richtlinien gefunden wurde.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 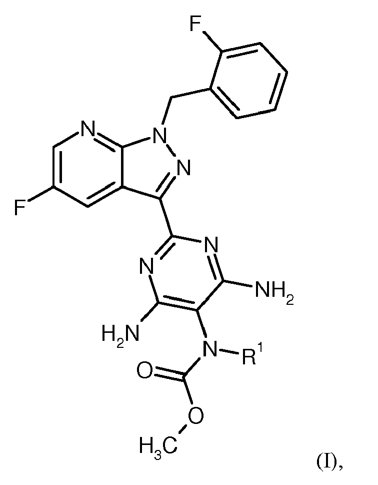 in welcher
R¹ für Wasserstoff oder (C₁-C₄)-Alkyl steht,
wobei (C₁-C₄)-Alkyl mit ein oder zwei Substituenten unabhängig voneinander ausgewählt aus der Gruppe Fluor und Trifluormethyl substituiert sein kann,
sowie ihre *N*-Oxide, Salze, Solvate, Salze der *N*-Oxide und Solvate der *N*-Oxide und Salze.

2. Verbindung der Formel (I) nach Anspruch 1, in welcher
R¹ für Wasserstoff oder Methyl steht,
wobei Methyl mit einem Substituenten Trifluormethyl substituiert sein kann,
sowie ihre Salze, Solvate und Solvate der Salze.

3. Verbindung der Formel (I) nach Anspruch 1 oder 2 ausgewählt aus der Gruppe:
Methyl-{4,6-diamino-2-[5-fluor-1-(2-fluorbenzyl)-1H-pyrazolo[3,4-b]pyridin-3-yl]pyrimidin-5-yl}carbamat
Methyl-{4,6-diamino-2-[5-fluor-1-(2-fluorbenzyl)-1H-pyrazolo[3,4-b]pyridin-3-yl]pyrimidin-5-yl}methylcarbamat
Methyl-{4,6-diamino-2-[5-fluor-1-(2-fluorbenzyl)-1H-pyrazolo[3,4-b]pyridin-3-yl]pyrimidin-5-yl}(2,2,2-trifluorethyl)carbamat
Methyl-{4,6-diamino-2-[5-fluor-1-(2-fluorbenzyl)-1H-pyrazolo[3,4-b]pyridin-3-yl]pyrimidin-5-yl}carbamat-Hydrochlorid
Methyl-{4,6-diamino-2-[5-fluor-1-(2-fluorbenzyl)-1H-pyrazolo[3,4-b]pyridin-3-yl]pyrimidin-5-yl}carbamat-Sulfat
Methyl-{4,6-diamino-2-[5-fluor-1-(2-fluorbenzyl)-1H-pyrazolo[3,4-b]pyridin-3-yl]pyrimidin-5-yl}carbamat-Phosphat
Methyl-{4,6-diamino-2-[5-fluor-1-(2-fluorbenzyl)-1H-pyrazolo[3,4-b]pyridin-3-yl]pyrimidin-5-yl}carbamat-Mesylat
Methyl-{4,6-diamino-2-[5-fluor-1-(2-fluorbenzyl)-1H-pyrazolo[3,4-b]pyridin-3-yl]pyrimidin-5-yl}carbamat-Ethan-1,2-disulfonat
Methyl-{4,6-diamino-2-[5-fluor-1-(2-fluorbenzyl)-1H-pyrazolo[3,4-b]pyridin-3-yl]pyrimidin-5-yl}carbamat-Maleat
Methyl-{4,6-diamino-2-[5-fluor-1-(2-fluorbenzyl)-1H-pyrazolo[3,4-b]pyridin-3-yl]pyrimidin-5-yl}carbamat-Nitrat.

4. Methyl-{4,6-diamino-2-[5-fluor-1-(2-fluorbenzyl)-1H-pyrazolo[3,4-b]pyridin-3-yl]pyrimidin-5-yl}carbamat gemäß Anspruch 1 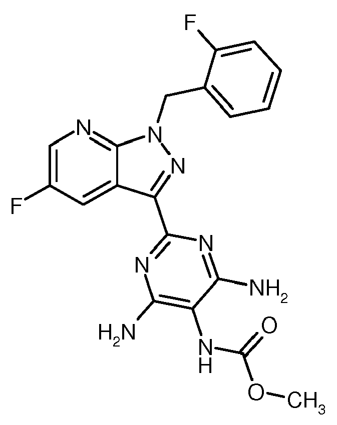 sowie dessn Salze, Solvate und Solvate der Salze.

5. Verfahren zur Herstellung von Verbindungen der Formel (I), wie in den Ansprüchen 1 bis 4 definiert, **dadurch gekennzeichnet, dass** man die Verbindung der Formel (II) 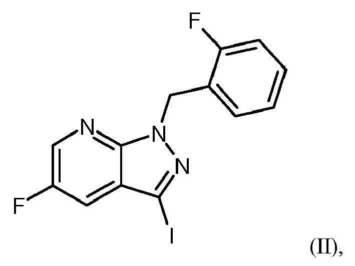
[A] in einem inerten Lösungsmittel in Gegenwart von Hexabutylzinn und einem geeigneten Palladiumkatalysator unter intermediärer Bildung einer Zinnspecies mit der Verbindung der Formel (III) 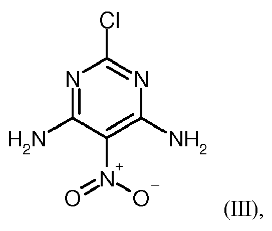 zur Verbindung der Formel (IV) 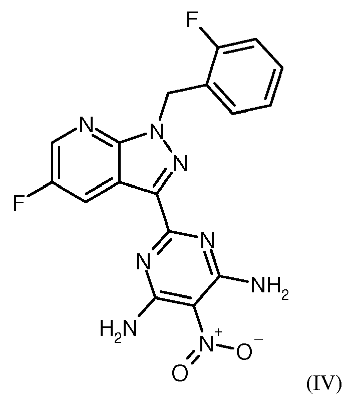 umsetzt, diese dann in einem inerten Lösungsmittel mit einem geeigneten Reduktionsmittel zur Verbindung der Formel (V) 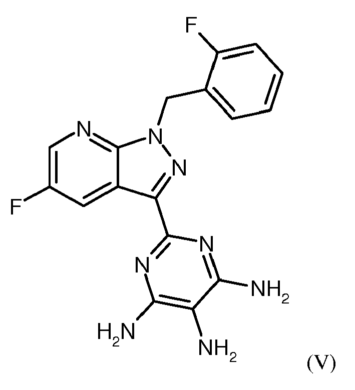 reduziert, diese im Anschluss in Gegenwart einer geeigneten Base mit oder ohne Lösungsmittel mit Chlorameisensäuremethylester zur Verbindung der Formel (I-A) 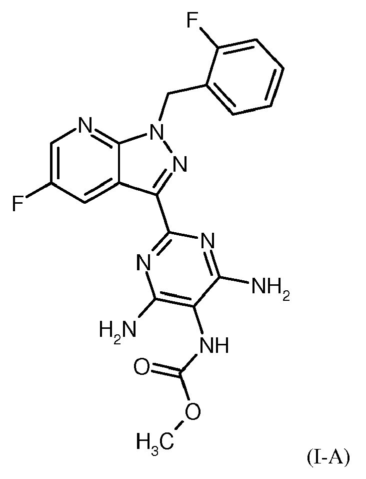 umsetzt,
oder
[B] die Verbindung der Formel (II) in einem inerten Lösungsmittel mit Kupfercyanid zur Verbindung der Formel (VI) 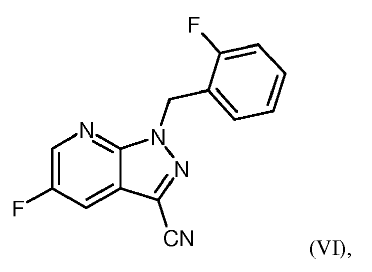 umsetzt, diese dann unter sauren Bedingungen in die Verbindung der Formel (VII) 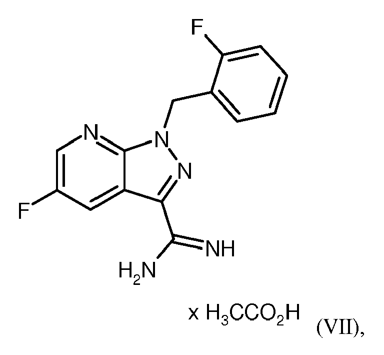 überführt, diese im Folgenden in einem inerten Lösungsmittel in Gegenwart einer geeigneten Base mit der Verbindung der Formel (VIII) 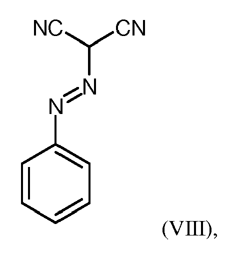 zur Verbindung der Formel (IX) 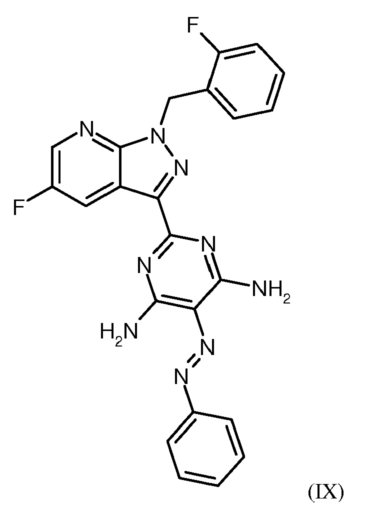 reagiert, und diese anschliessend in einem inertem Lösungsmittel in Gegenwart eines geeigneten Reduktionsmittels zur Verbindung (V) reduziert, und diese im Folgenden nach Verfahren [A] weiter zu Verbindung (I-A) umsetzt,
oder
[C] die Verbindung der Formel (I-A) in einem inerten Lösungsmittel in Gegenwart einer geeigneten Base mit einer Verbindung der Formel (X) 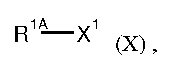 in welcher
R^{1A} für (C₁-C₄)-Alkyl steht,
wobei (C₁-C₄)-Alkyl mit ein oder zwei Substituenten unabhängig voneinander ausgewählt aus der Gruppe Fluor und Trifluormethyl substituiert sein kann,
und
X¹ für eine Abgangsgruppe, wie beispielsweise Halogen, insbesondere Brom oder Iod, Trichlormethansulfonat, Mesylat oder Tosylat steht
zu einer Verbindung der Formel (I-B) 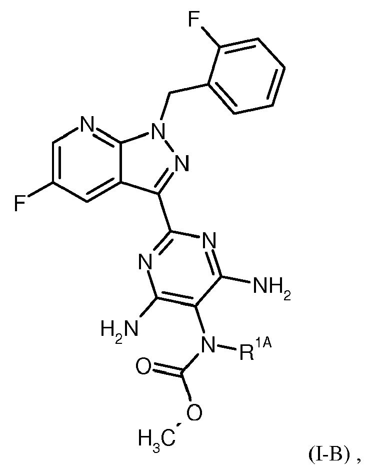
in welcher R^{1A} die zuvor angegebene Bedeutung hat,
umsetzt,
und die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

6. Verbindung der Formel (I), wie in einem der Ansprüche 1 bis 4 definiert, zur Verwendung in einem Verfahren zur Behandlung und/oder Prophylaxe von Krankheiten.

7. Verbindung der Formel (I), wie in einem der Ansprüche 1 bis 4 definiert, zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Verwendung einer Verbindung der Formel (I), wie in einem der Ansprüche 1 bis 4 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

9. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

10. Arzneimittel enthaltend eine Verbindung der Formel (I), wie in einem der Ansprüche 1 bis 4 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

11. Arzneimittel nach Anspruch 9 oder 10 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 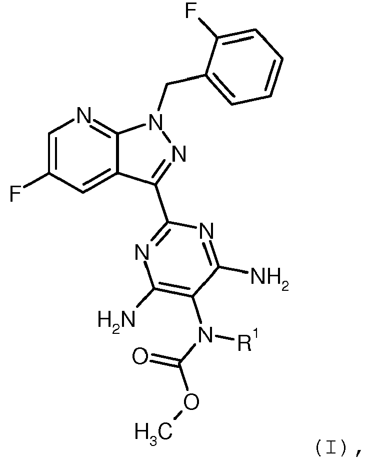 in which
R¹ represents hydrogen or (C₁-C₄)-alkyl,
where (C₁-C₄)-alkyl may be substituted by one or two substituents independently of one another selected from the group consisting of fluorine and trifluoromethyl,
and their *N*-oxides, salts, solvates, salts of the *N*-oxides and solvates of the *N*-oxides and salts.

2. Compound of the formula (I) according to Claim 1 in which
R¹ represents hydrogen or methyl,
where methyl may be substituted by a trifluoromethyl substituent,
and their salts, solvates and solvates of the salts.

3. Compound of the formula (I) according to Claim 1 or 2, selected from the group consisting of:
methyl {4,6-diamino-2-[5-fluoro-1-(2-fluorobenzyl)-1H-pyrazolo[3,4-b]pyridin-3-yl]pyrimidin-5-yl}carbamate
methyl {4,6-diamino-2-[5-fluoro-1-(2-fluorobenzyl)-1H-pyrazolo[3,4-b]pyridin-3-yl]pyrimidin-5-yl}methylcarbamate
methyl {4,6-diamino-2-[5-fluoro-1-(2-fluorobenzyl)-1H-pyrazolo[3,4-b]pyridin-3-yl]pyrimidin-5-yl}(2,2,2-trifluoroethyl)carbamate
methyl {4,6-diamino-2-[5-fluoro-1-(2-fluorobenzyl)-1H-pyrazolo[3,4-b]pyridin-3-yl]pyrimidin-5-yl}carbamate hydrochloride
methyl {4,6-diamino-2-[5-fluoro-1-(2-fluorobenzyl)-1H-pyrazolo[3,4-b]pyridin-3-yl]pyrimidin-5-yl}carbamate sulphate
methyl {4,6-diamino-2-[5-fluoro-1-(2-fluorobenzyl)-1H-pyrazolo[3,4-b]pyridin-3-yl]pyrimidin-5-yl}carbamate phosphate
methyl {4,6-diamino-2-[5-fluoro-1-(2-fluorobenzyl)-1H-pyrazolo[3,4-b]pyridin-3-yl]pyrimidin-5-yl}carbamate mesylate
methyl {4,6-diamino-2-[5-fluoro-1-(2-fluorobenzyl)-1H-pyrazolo[3,4-b]pyridin-3-yl]pyrimidin-5-yl}carbamate ethane-1,2-disulphonate
methyl {4,6-diamino-2-[5-fluoro-1-(2-fluorobenzyl)-1H-pyrazolo[3,4-b]pyridin-3-yl]pyrimidin-5-yl}carbamate maleate
methyl {4,6-diamino-2-[5-fluoro-1-(2-fluorobenzyl)-1H-pyrazolo[3,4-b]pyridin-3-yl]pyrimidin-5-yl}carbamate nitrate.

4. Methyl {4,6-diamino-2-[5-fluoro-1-(2-fluorobenzyl)-1H-pyrazolo[3,4-b]pyridin-3-yl]pyrimidin-5-yl}carbamate according to Claim 1 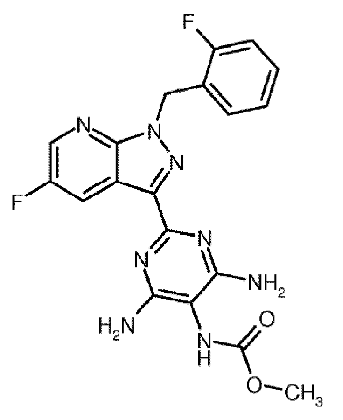 and its salts, solvates and solvates of the salts.

5. Process for preparing compounds of the formula (I) as defined in any of Claims 1 to 4, **characterized in that** the compound of the formula (II) 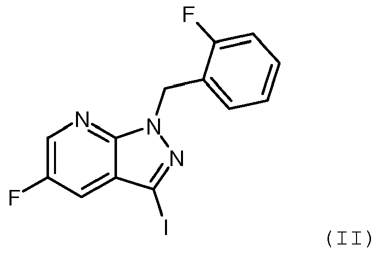
[A] is reacted in an inert solvent in the presence of hexabutyltin and a suitable palladium catalyst with intermediate formation of a tin species with the compound of the formula (III) 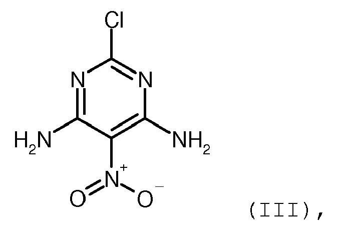 to give the compound of the formula (IV) 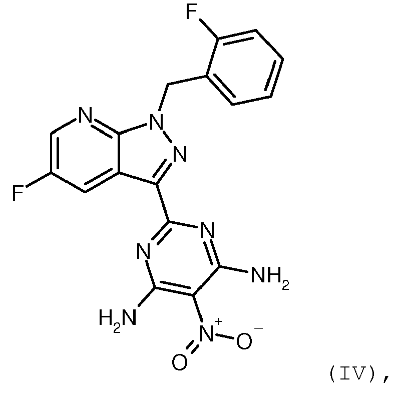 this is then reduced in an inert solvent with a suitable reducing agent to give the compound of the formula (V) 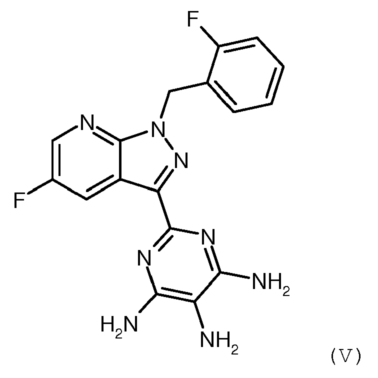 and this is then reacted in the presence of a suitable base in the presence or absence of a solvent with methyl chloroformate to give the compound of the formula (I-A) 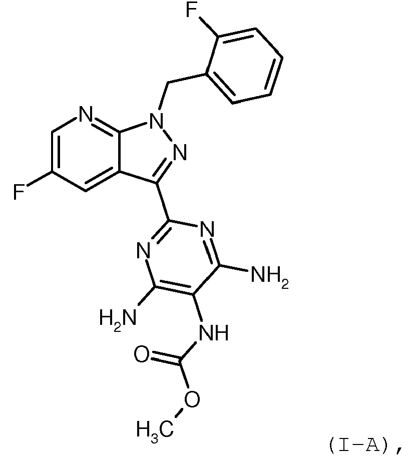 or
[B] the compound of the formula (II) is reacted in an inert solvent with copper cyanide to give the compound of the formula (VI) 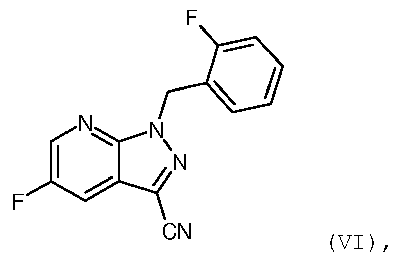 this is then, under acidic conditions, converted into the compound of the formula (VII) 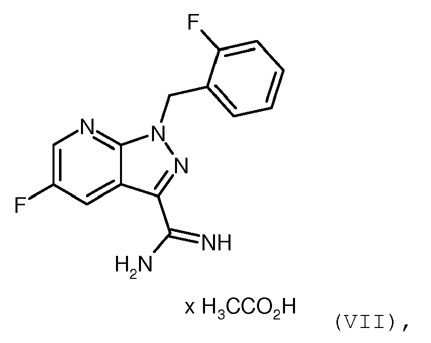 this is subsequently reacted in an inert solvent in the presence of a suitable base with the compound of the formula (VIII) 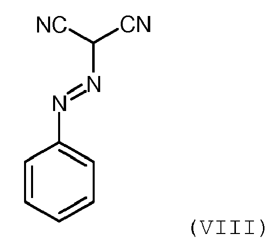 to give the compound of the formula (IX) 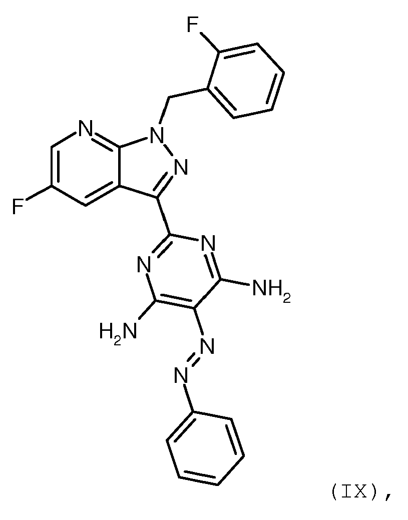 and this is then reduced in an inert solvent in the presence of a suitable reducing agent to give the compound (V), and this is subsequently reacted further according to process [A] to give compound (I-A),
or
[C] the compound of the formula (I-A) is reacted in an inert solvent in the presence of a suitable base with a compound of the formula (X) 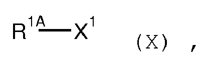 in which
R^{1A} represents (C₁-C₄)-alkyl,
where (C₁-C₄)-alkyl may be substituted by one or two substituents independently of one another selected from the group consisting of fluorine and trifluoromethyl,
and
X¹ represents a leaving group such as, for example, halogen, in particular bromine or iodine, trichloromethanesulphonate, mesylate or tosylate
to give a compound of the formula (I-B) 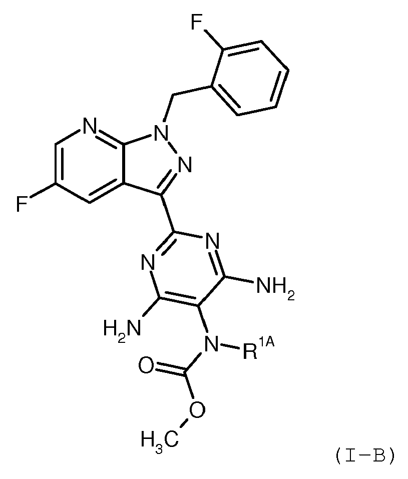
in which R^{1A} has the meaning given above,
and the resulting compounds of the formulae (I-A) and (I-B) are, where appropriate, converted with the appropriate (i) solvents and/or (ii) acids or bases into their solvates, salts and/or solvates of the salts.

6. Compound of the formula (I) as defined in any of Claims 1 to 4 for use in a method for the treatment and/or prophylaxis of diseases.

7. Compound of the formula (I) as defined in any of Claims 1 to 4 for use in a method for the treatment and/or prophylaxis of heart failure, angina pectoris, hypertension, pulmonary hypertension, ischaemias, vascular disorders, kidney failure, thromboembolic disorders, fibrotic disorders and arteriosclerosis.

8. Use of a compound of the formula (I) as defined in any of Claims 1 to 4 for the preparation of a medicament for the treatment and/or prophylaxis of heart failure, angina pectoris, hypertension, pulmonary hypertension, ischaemias, vascular disorders, kidney failure, thromboembolic disorders, fibrotic disorders and arteriosclerosis.

9. Medicament comprising a compound of the formula (I) as defined in any of Claims 1 to 4 in combination with an inert, non-toxic, pharmaceutically suitable excipient

10. Medicament comprising a compound of the formula (I) as defined in any of Claims 1 to 4 in combination with a further active compound selected from the group consisting of organic nitrates, NO donors, cGMP-PDE inhibitors, agents having antithrombotic activity, agents lowering blood pressure, and agents altering lipid metabolism.

11. Medicament according to Claim 9 or 10 for the treatment and/or prophylaxis of heart failure, angina pectoris, hypertension, pulmonary hypertension, ischaemias, vascular disorders, kidney failure, thromboembolic disorders, fibrotic disorders and arteriosclerosis.

## Revendications

1. Composé de formule (I) 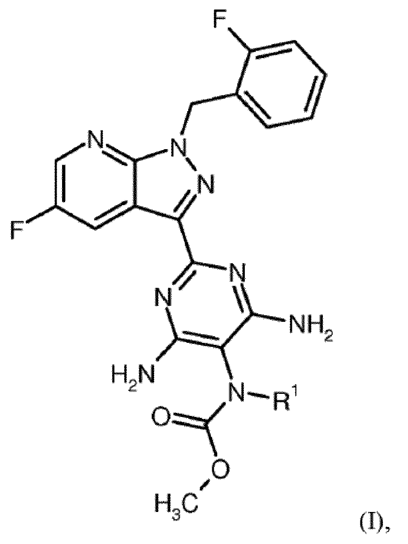 dans lequel
R¹ représente hydrogène ou alkyle en (C₁-C₄), alkyle en (C₁-C₄) pouvant être substitué avec un ou deux substituants choisis indépendamment l'un de l'autre dans le groupe constitué par fluor et trifluorométhyle,
ainsi que leurs N-oxydes, sels, solvates, sels des N-oxydes et solvates des N-oxydes et des sels.

2. Composé de formule (I) selon la revendication 1, dans lequel
R¹ représente hydrogène ou méthyle, méthyle pouvant être substitué avec un substituant trifluorométhyle,
ainsi que leurs sels, solvates et solvates des sels.

3. Composé de formule (I) selon la revendication 1 ou 2, choisi dans le groupe constitué par :
le {4,6-diamino-2-[5-fluoro-1-(2-fluorobenzyl)-1H-pyrazolo[3,4-b]pyridin-3-yl]pyrimidin-5-yl}carbamate de méthyle
le {4,6-diamino-2-[5-fluoro-1-(2-fluorobenzyl)-1H-pyrazolo[3,4-b]pyridin-3-yl]pyrimidin-5-yl}méthylcarbamate de méthyle
le {4,6-diamino-2-[5-fluoro-1-(2-fluorobenzyl)-1H-pyrazolo[3,4-b]pyridin-3-yl]pyrimidin-5-yl}(2,2,2-trifluoroéthyl)carbamate de méthyle
le chlorhydrate de {4,6-diamino-2-[5-fluoro-1-(2-fluorobenzyl)-1H-pyrazolo[3,4-b]pyridin-3-yl]pyrimidin-5-yl}carbamate de méthyle
le sulfate de {4,6-diamino-2-[5-fluoro-1-(2-fluorobenzyl)-1H-pyrazolo[3,4-b]pyridin-3-yl]pyrimidin-5-yl}carbamate de méthyle
le phosphate de {4,6-diamino-2-[5-fluoro-1-(2-fluorobenzyl)-1H-pyrazolo[3,4-b]pyridin-3-yl]pyrimidin-5-yl}carbamate de méthyle
le mésylate de {4,6-diamino-2-[5-fluoro-1-(2-fluorobenzyl)-1H-pyrazolo[3,4-b]pyridin-3-yl]pyrimidin-5-yl}carbamate de méthyle
l'éthane-1,2-disulfonate de {4,6-diamino-2-[5-fluoro-1-(2-fluorobenzyl)-1H-pyrazolo[3,4-b]pyridin-3-yl]pyrimidin-5-yl}carbamate de méthyle
le maléate de {4,6-diamino-2-[5-fluoro-1-(2-fluorobenzyl)-1H-pyrazolo[3,4-b]pyridin-3-yl]pyrimidin-5-yl}carbamate de méthyle
le nitrate de {4,6-diamino-2-[5-fluoro-1-(2-fluorobenzyl)-1H-pyrazolo[3,4-b]pyridin-3-yl]pyrimidin-5-yl}carbamate de méthyle.

4. {4,6-Diamino-2-[5-fluoro-1-(2-fluorobenzyl)-1H-pyrazolo[3,4-b]pyridin-3-yl]pyrimidin-5-yl}carbamate de méthyle selon la revendication 1, 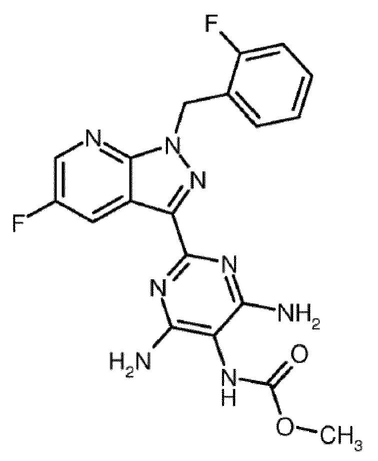 ainsi que ses sels, solvates et solvates des sels.

5. Procédé de fabrication de composés de formule (I), tels que définis dans les revendications 1 à 4, **caractérisé en ce que** le composé de formule (II) 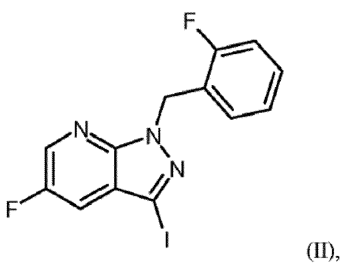
[A] est mis en réaction dans un solvant inerte en présence d'hexabutylétain et d'un catalyseur de palladium approprié avec formation intermédiaire d'une espèce d'étain avec le composé de formule (III) 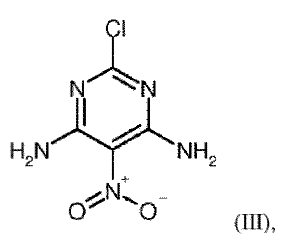 pour former le composé de formule (IV) 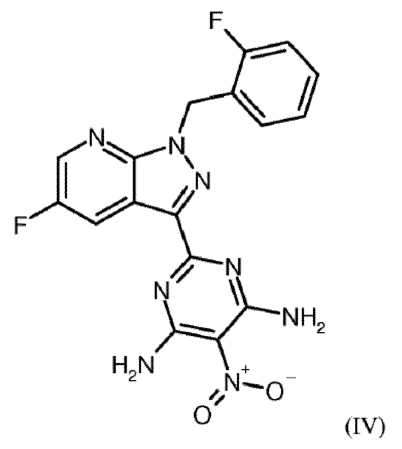 puis celui-ci est réduit dans un solvant inerte avec un réducteur approprié pour former le composé de formule (V) 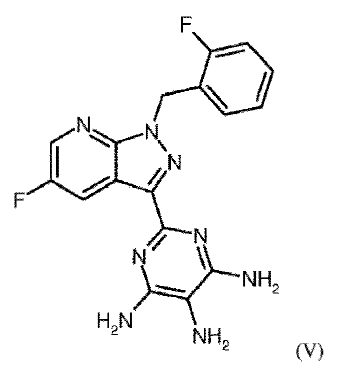 puis celui-ci est mis en réaction en présence d'une base appropriée avec ou sans solvant avec l'ester méthylique de l'acide chloroformique pour former le composé de formule (I-A) 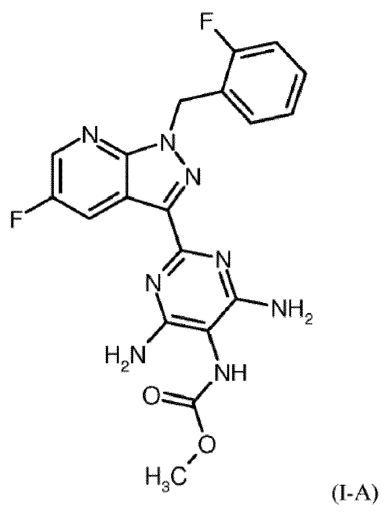 ou
[B] le composé de formule (II) est mis en réaction dans un solvant inerte avec du cyanure de cuivre pour former le composé de formule (VI) 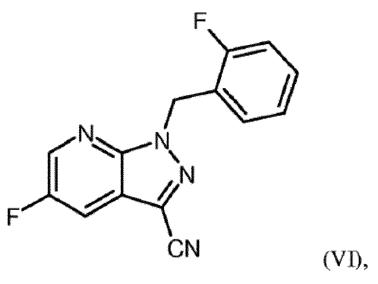 puis celui-ci est transformé en conditions acides en le composé de formule (VII) 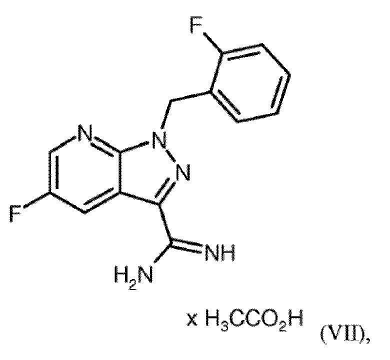 puis celui-ci est mis en réaction dans un solvant inerte en présence d'une base appropriée avec le composé de formule (VIII) 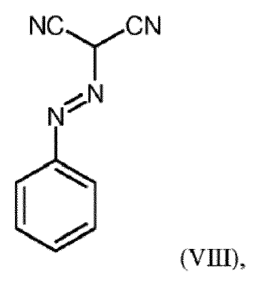 pour former le composé de formule (IX) 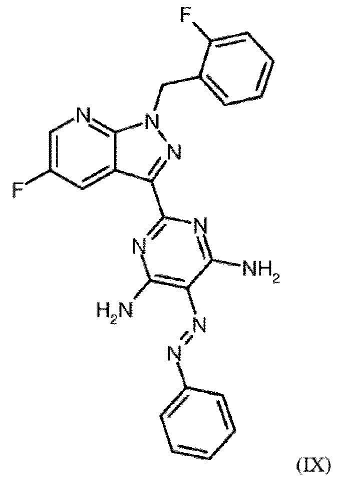 puis celui-ci est réduit dans un solvant inerte en présence d'un réducteur approprié en le composé (V), et celui-ci est ensuite transformé en le composé (I-a) selon le procédé [A],
ou
[C] le composé de formule (I-A) est mis en réaction dans un solvant inerte en présence d'une base appropriée avec un composé de formule (X) 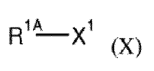 dans lequel
R^{1A} représente alkyle en (C₁-C₄), alkyle en (C₁-C₄) pouvant être substitué avec un ou deux substituants choisis indépendamment l'un de l'autre dans le groupe constitué par fluor et trifluorométhyle,
et
X¹ représente un groupe partant, tel que par exemple halogène, notamment brome ou iode, trichlorométhanesulfonate, mésylate ou tosylate,
pour former un composé de formule (I-B) 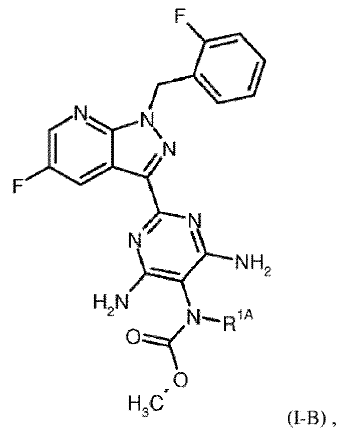 dans lequel R^{1A} a la signification indiquée précédemment,
et les composés de formule (I-A) et (I-B) résultants sont éventuellement transformés avec (i) les solvants et/ou (ii) les acides ou les bases appropriés en leurs solvates, sels et/ou solvates des sels.

6. Composé de formule (I), tel que défini dans l'une quelconque des revendications 1 à 4, destiné à une utilisation dans un procédé de traitement et/ou de prophylaxie de maladies.

7. Composé de formule (I), tel que défini dans l'une quelconque des revendications 1 à 4, destiné à une utilisation dans un procédé de traitement et/ou de prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

8. Utilisation d'un composé de formule (I), tel que défini dans l'une quelconque des revendications 1 à 4, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

9. Médicament contenant un composé de formule (I), tel que défini dans l'une quelconque des revendications 1 à 4, en combinaison avec un adjuvant inerte, non toxique, pharmaceutiquement approprié.

10. Médicament contenant un composé de formule (I), tel que défini dans l'une quelconque des revendications 1 à 4, en combinaison avec un autre agent actif choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

11. Médicament selon la revendication 9 ou 10, pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.
